# EUROPEAN PATENT APPLICATION

(11) **EP 4 371 983 A1**
(43) Date of publication of application: **22.05.2024**
(21) Application number: 22842392.7
(22) Date of filing: 11.07.2022
(51) Int. Cl.: C07D 401/14, A61K 31/506, A61P 35/00, C07D 401/12

(54) **NOVEL PYRIMIDINE-2,4-DIAMINE DERIVATIVES, PREPARATION METHOD THEREFOR, AND PHARMACEUTICAL COMPOSITION CONTAINING SAME AS ACTIVE INGREDIENT FOR PREVENTION OR TREATMENT OF CANCER**

(30) Priority: 13.07.2021 KR 20210091308; 08.07.2022 KR 20220084115
(71) Applicant: Korea Research Institute of Chemical Technology, Daejeon 34114 (KR)
(72) Inventor: LEE, Kwangho, Daejeon 34114 (KR); DUGGIRALA, Krishna Babu, Daejeon 34114 (KR); CHOI, Gildon, Daejeon 34114 (KR); LEE, Yujin, Gimcheon-si Gyeongsangbuk-do 39589 (KR); CHAE, Chong Hak, Daejeon 34114 (KR); JUNG, Myoung Eun, Daejeon 34114 (KR); CHO, Byoung Chul, Seoul 04425 (KR)
(74) Representative: HGF
(86) International application number: PCT/KR2022/010023
(87) International publication number: WO 2023/287130

(57) **Abstract**

The present invention relates to a pyrimidine-2,4-diamine derivative, a method for preparing the same, and a pharmaceutical composition for preventing or treating cancer, comprising the same as an active ingredient. The pyrimidine-2,4-diamine derivative exhibits a high inhibitory ability against EGFR and HER2 mutations, and thus can be advantageously used in the treatment of cancer in which EGFR and HER2 mutations have occurred. In addition, the pyrimidine-2,4-diamine derivative exhibits a significant synergistic effect when administered in combination, and thus may be advantageously used in combination therapy.

## Description

### Technical Field

The present invention relates to a pyrimidine-2,4-diamine derivative, a method for preparing the same, and a pharmaceutical composition for preventing or treating cancer, comprising the same as an active ingredient.

### Background Art

The development of cancer is related to various environmental factors, including chemicals, radiation, and viruses, as well as changes in oncogenes, tumor suppressor genes, and genes related to apoptosis and DNA repair. Recently, with the understanding of these molecular mechanisms of cancer, a new treatment method, targeted anticancer therapy, has become possible.

Targeted therapeutic agents are generally made to exert their effects by targeting molecules that cancer cells have characteristically. Molecular targets include genes related to the signal transduction pathway, angiogenesis, matrix, cell cycle regulator, and apoptosis of cancer cells. Currently, important targeted therapeutic agents used in treatment include 'signal transduction pathway inhibitors' and 'angiogenesis inhibitors', including tyrosine kinase inhibitors.

Protein tyrosine kinases have been found to play an important role in many malignant tumors. In particular, it is known that epidermal growth factor receptor (EGFR), a receptor tyrosine kinase of the erbB family, is abnormally activated in many epithelial cell tumors, including non-small cell lung carcinoma (NSCLC), breast cancer, glioma, squamous cell carcinoma of the head and neck, large intestine cancer, rectal adenocarcinoma, head and neck cancer, stomach cancer, and prostate cancer, and activation of the EGFR-tyrosine kinase causes continuous cell proliferation, invasion of surrounding tissues, distant metastasis, blood vessel formation, and increases cell survival.

Specifically, the EGFR is one of the ErbB tyrosine kinase receptors family (EGFR, HER2, ErbB3, ErbB4), and is a transmembrane tyrosine kinase that has an intracellular domain including an extracellular ligand-binding domain and a tyrosine kinase domain. When a ligand binds to a homodimer or heterodimer receptor, intracellular tyrosine kinase is activated, and the signal stimulated by EGFR activates signal transduction pathway of phosphatidylinositol 3-kinase (PI3K)/AKT/mTOR, RAS/RAF/MAPK, and JAK/STAT (Nat Rev Cancer 2007;7:169-81).

In particular, EGFR is overexpressed in more than half of non-small cell lung cancer (NSCLC), and many studies have been conducted as a target for treatment. EGFR TKIs (tyrosine kinase inhibitors) that inhibit EGFR tyrosine kinase activity have been developed, and representative drugs include gefitinib (IRESSA^{™}), erlotinib (TARCEVA^{™}), and lapatinib (TYKERB^{™}, TYVERB^{™}).

Meanwhile, in 2004, it was reported that activating mutations in EGFR are correlated with the response to gefitinib therapy in non-small-cell lung cancer (NSCLC) (Science [2004] Vol.304, 1497-500 and New England Journal of Medicine [2004] Vol. 350, 2129-2139).

Specifically, the EGFR mutations are largely divided into sensitizing mutations and resistant mutations. The deletion of exon 19 and the L858R point mutation in exon 21 are the most important sensitizing mutations, accounting for approximately 85 to 90%, and the exon 19 del mutation is known to have better sensitizing to TKI. On the other hand, the T790M point mutation in exon 20 is the most important resistant mutation and is known to be found in more than 50% of patients with acquired resistance (Clin Cancer Res 2006;12:6494-6501.).

Somatic mutations identified to date include point mutations (e.g., L858R, G719S, G719C, G719A, L861Q) in which a single nucleotide residue is modified within the expressed protein, as well as in-frame deletions in exon 19 or insertions in exon 20 (Fukuoka et al. JCO 2003; Kris et al JAMA 2003 and Shepherd et al NEJM 2004).

Despite the initial clinical effectiveness of gefitinib/erlotinib in NSCLC patients with EGFR mutations, most patients eventually develop progressive cancer while receiving therapy with these agents. Initial studies of relapsed specimens identified a secondary EGFR mutation, T790M, which renders gefitinib and erlotinib ineffective inhibitors of EGFR kinase activity (Kobayashi et al., NEJM 2005 and Pao et al., PLOS Medicine 2005). Subsequent studies demonstrated that the EGFR T790M mutation was found in approximately 50% (24/48) of tumors derived from patients who acquired resistance to gefitinib or erlotinib (Kosaka et al CCR 2006; Balak et al CCR 2006 and Engelman et al Science 2007). These secondary genetic modifications occur at positions similar to 'gatekeeper' residues and their associated secondary resistance alleles in patients treated with kinase inhibitors (e.g., T315I in ABL in imatinib-resistant CML).

It has long been known that the EGFR mutations, EGFR_del19 or EGFR_L858R, are a major cause of non-small cell lung cancer and head and neck cancer, and drugs for treating them, Iressa and Tarceva, have been developed and are currently used clinically. However, when these drugs were used in patients, an acquired resistance, in which secondary mutations of EGFR occurred based on the structure of the drug, was observed, and it was also revealed that this was the main cause of actual drug resistance. When first-generation EGFR inhibitors are used for an average of 10 months, an acquired resistance called the T790M mutation located in the gatekeeper of EGFR kinase occurs, making the first-generation EGFR inhibitors ineffective. In other words, EGFR_del19_T790M or EGFR_L858R_T790M double mutation occurs, making conventional therapeutic agents ineffective.

Based on these facts, the need to develop second- and third-generation drugs with excellent efficacy and new structures has emerged.

Over the past 10 years, various third-generation drugs that have shown effectiveness against EGFR T790M double mutations include AZD9291 (osimertinib, Tagrisso) from AstraZeneca, a multinational pharmaceutical company. However, it has been reported that resistance to AZD9291 develops again in about 10 months and the efficacy of AZD9291 is lost. In particular, it has been reported that resistance occurs due to a triple mutation including C797S (Thress et al., Nature Medicine 2015).

Accordingly, there is a need for the development of inhibitors that exhibit inhibition against relatively low WT EGFR and, at the same time, higher inhibition against specific activated or various resistant mutant forms of EGFR.

The EGFR TKIs (tyrosine kinase inhibitors) gefitinib, erlotinib, and afatinib are approved therapeutic agents for non-small cell lung cancer with activating mutations in the EGFR kinase. However, there is a problem in that resistance to these drugs develops rapidly and the T790M mutation in the ATP site of the receptor occurs frequently. Accordingly, the recently developed irreversible, mutation-selective inhibitor exhibits high activity against the T790M mutation, but its efficacy can be neutralized by the acquired mutation of C797, the cysteine residue that forms the key covalent bond.

Intracellular human EGFR2, known as HER2/neu or ErbB2, is a tyrosine kinase receptor belonging to the human epidermal growth factor receptor (HER/EGFR/ERBB) family. It is normally involved in signal transduction pathways and induces cell growth and differentiation. HER2 shows very high structural similarity to the other three EGFR family members (EGFR, HER3, and HER4).

However, unlike other anticancer targets, in the case of HER2 (human epidermal growth factor 2), the ligand that binds to it is not yet known, and it partners with other HER receptors that bind to the ligand, forms a heterodimer, and is involved in cell cycle increase, cell proliferation regulation, differentiation and survival through various signal pathways. In the case of antibodies targeting Her2, antibody therapeutic agents of the IgG2 type have been developed and are commercially available. The main therapeutic action is not antibody-dependent cellular cytotoxicity (ADCC) or complement-dependent cytotoxicity (CDC), but is neutralizing activity due to the antibody.

Accordingly, while the present inventors were trying to develop a cancer therapeutic agent that inhibits EGFR multiple mutations, it was found that the pyrimidine-2,4-diamine derivative according to the present invention exhibits relatively low inhibition against wild type EGFR and a high inhibitory ability against an EGFR mutation and a HER2 mutation, and thus it can be advantageously used in the prevention or treatment of cancer. Based on the above, the present inventors completed the present invention.

### Detailed Description of Invention

### Technical Problem

An object of the present invention is to provide a pyrimidine-2,4-diamine derivative.

Another object of the present invention is to provide a method for preparing a pyrimidine-2,4-diamine derivative.

Another object of the present invention is to provide a pharmaceutical composition for preventing or treating cancer, containing a pyrimidine-2,4-diamine derivative as an active ingredient.

Another object of the present invention is to provide a health functional food for preventing or improving cancer, containing a pyrimidine-2,4-diamine derivative as an active ingredient.

### Solution to Problem

In order to achieve the above object, according to one aspect of the present invention, there is provided a compound represented by Formula 1 below, a stereoisomer thereof, a solvate thereof, a hydrate thereof, or a pharmaceutically acceptable salt thereof: 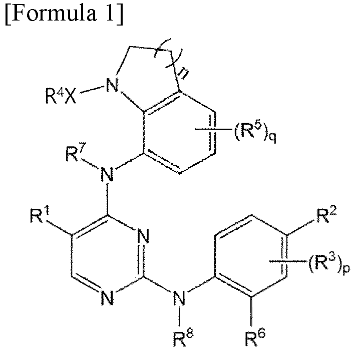 in Formula 1 above,
n is an integer from 0 to 3;
X is sulfonyl or carbonyl;
R¹ is hydrogen, halogen, nitrile, a straight chain or branched chain C₁₋₁₀ alkyl unsubstituted or substituted with one or more halogens, carboxyl, or a straight chain or branched chain C₁₋₁₀ alkoxycarbonyl;
R² is -ORₐ or -NR_{b1}R_{b2},
Rₐ is -(CH₂)ₘ-NR_{b1}R_{b2}, wherein m is an integer from 1 to 3,
R_{b1} and R_{b2} are each independently hydrogen, an unsubstituted or substituted straight chain or branched chain C₁₋₁₀ alkyl, or R_{b1} and R_{b2} are taken together with the nitrogen to which they are attached to form an unsubstituted or substituted 3 to 7-membered heterocycloalkyl, wherein the substituent of the substituted straight chain or branched chain C₁₋₁₀ alkyl may be - NR_{c1}R_{c2}, and the substituent of the substituted 3 to 7-membered heterocycloalkyl may be - NR_{c1}R_{c2} or a straight chain or branched chain C₁₋₁₀ alkyl, and
R_{c1} and R_{c2} are each independently hydrogen, a straight chain or branched chain C₁₋₁₀ alkyl, or R_{c1} and R_{c2} are taken together with the nitrogen to which they are attached to form an unsubstituted or substituted 3 to 7-membered heterocycloalkyl, wherein the substituent of the substituted 3 to 7-membered heterocycloalkyl may be a straight chain or branched chain C₁₋₁₀ alkyl; and
R³, R⁵ and R⁶ are each independently hydrogen, halogen, a straight chain or branched chain C₁₋₁₀alkoxy, or a straight chain or branched chain C₁₋₁₀ alkyl unsubstituted or substituted with one or more halogens;
R⁴ is -NH₂, a straight chain or branched chain C₁₋₁₀ alkyl, or C₃₋₇ cycloalkyl;
R⁷ and R⁸ are each independently hydrogen or a straight chain or branched chain C₁₋₁₀ alkyl;
n is an integer from 0 to 3; and
p and q are each independently an integer from 1 to 3.

In addition, the present invention provides a method for preparing the compound represented by Formula 1 according to claim 1, comprising: reacting a compound represented by Formula 2 with a compound represented by Formula 3 to prepare a compound represented by Formula 1, as shown in Reaction Scheme 1 below: 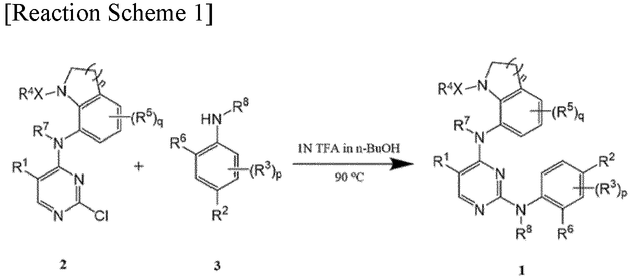 in Reaction Scheme 1 above, X, R¹, R², R³, R⁴, R⁵, R⁶, R⁷, R⁸, n, p and q are as defined in Formula 1 according to claim 1.

According to another aspect of the present invention, there is provided a pharmaceutical composition for preventing or treating cancer, containing the compound represented by Formula 1, a stereoisomer thereof, a solvate thereof, a hydrate thereof, or a pharmaceutically acceptable salt thereof as an active ingredient.

According to another aspect of the present invention, there is provided a health functional food for preventing or improving cancer, containing the compound represented by Formula 1, a stereoisomer thereof, a solvate thereof, a hydrate thereof, or a pharmaceutically acceptable salt thereof as an active ingredient.

According to another aspect of the present invention, there is provided a method for preventing or treating cancer, comprising: administering a pharmaceutical composition or a health functional food containing the compound represented by Formula 1, a stereoisomer thereof, a solvate thereof, a hydrate thereof, or a pharmaceutically acceptable salt thereof as an active ingredient to a subject in need thereof.

According to another aspect of the present invention, there is provided a use of a pharmaceutical composition or a health functional food containing the compound represented by Formula 1, a stereoisomer thereof, a solvate thereof, a hydrate thereof, or a pharmaceutically acceptable salt thereof for the prevention or treatment of cancer.

### Effects of Invention

The pyrimidine-2,4-diamine derivative of the present invention exhibits a high inhibitory ability against EGFR and HER2 mutations, and thus can be advantageously used in the treatment of cancer in which EGFR and HER2 mutations have occurred. In addition, the pyrimidine-2,4-diamine derivative exhibits a significant synergistic effect when administered in combination, and thus can be advantageously used in combination therapy.

### Best Mode for Carrying out the Invention

Hereinafter, the present invention will be described in detail.

In one aspect of the present invention, there is provided a compound represented by Formula 1 below, a stereoisomer thereof, a solvate thereof, a hydrate thereof, or a pharmaceutically acceptable salt thereof 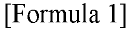 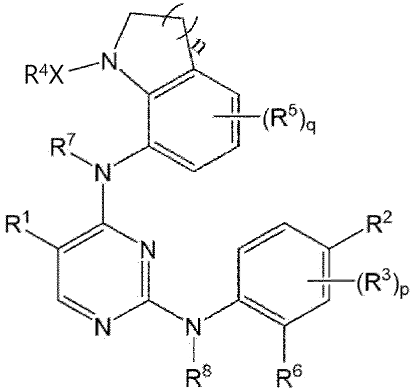 in Formula 1 above,
X is sulfonyl or carbonyl;
R¹ is hydrogen, halogen, nitrile, a straight chain or branched chain C₁₋₁₀ alkyl unsubstituted or substituted with one or more halogens, carboxyl, or a straight chain or branched chain C₁₋₁₀alkoxycarbonyl;
R² is -ORₐ or -NR_{b1}R_{b2},
Rₐ is -(CH₂)ₘ-NR_{b1}R_{b2}, wherein m is an integer from 1 to 3,
R_{b1} and R_{b2} are each independently hydrogen, an unsubstituted or substituted straight chain or branched chain C₁₋₁₀ alkyl, or R_{b1} and R_{b2} are taken together with the nitrogen to which they are attached to form an unsubstituted or substituted 3 to 7-membered heterocycloalkyl, wherein the substituent of the substituted straight chain or branched chain C₁₋₁₀ alkyl may be - NR_{c1}R_{c2}, and the substituent of the substituted 3 to 7-membered heterocycloalkyl may be - NR_{c1}R_{c2} or a straight chain or branched chain C₁₋₁₀ alkyl, and
R_{c1} and R_{c2} are each independently hydrogen, a straight chain or branched chain C₁₋₁₀ alkyl, or R_{c1} and R_{c2} are taken together with the nitrogen to which they are attached to form an unsubstituted or substituted 3 to 7-membered heterocycloalkyl, wherein the substituent of the substituted 3 to 7-membered heterocycloalkyl may be a straight chain or branched chain C₁₋₁₀ alkyl; and
R³, R⁵ and R⁶ are each independently hydrogen, halogen, a straight chain or branched chain C₁₋₁₀ alkoxy, or a straight chain or branched chain C₁₋₁₀ alkyl unsubstituted or substituted with one or more halogens;
R⁴ is -NH₂, a straight chain or branched chain C₁₋₁₀ alkyl, or C₃₋₇ cycloalkyl;
R⁷ and R⁸ are each independently hydrogen or a straight chain or branched chain C₁₋₁₀ alkyl;
n is an integer from 0 to 3; and
p and q are each independently an integer from 1 to 3.

In addition, R¹ may be hydrogen, halogen, a straight chain or branched chain C₁₋₃ alkyl unsubstituted or substituted with one or more halogens, or a straight chain or branched chain C₁₋₆alkoxycarbonyl;
R² may be -NR_{b1}R_{b2},
R_{b1} and R_{b2} may be each independently hydrogen, an unsubstituted or substituted straight chain or branched chain C₁₋₃ alkyl, or R_{b1} and R_{b2} may be taken together with the nitrogen to which they are attached to form an unsubstituted or substituted 5 to 6-membered heterocycloalkyl, wherein the substituent of the substituted straight chain or branched chain C₁₋₃ alkyl may be -NR_{c1}R_{c2}, and the substituent of the substituted 5 to 6-membered heterocycloalkyl may be -NR_{c1}R_{c2} or a straight chain or branched chain C₁₋₃ alkyl, and
R_{c1} and R_{c2} may be each independently hydrogen, a straight chain or branched chain C₁₋₃ alkyl, or R_{c1} and R_{c2} may be taken together with the nitrogen to which they are attached to form an unsubstituted or substituted 5 to 6-membered heterocycloalkyl, wherein the substituent of the substituted 5 to 6-membered heterocycloalkyl may be a straight chain or branched chain C₁₋₃ alkyl; and
R³ and R⁵ may be each independently hydrogen, or a straight chain or branched chain C₁₋₃ alkyl unsubstituted or substituted with one or more halogens;
R⁴ may be -NH₂, a straight chain or branched chain C₁₋₃ alkyl, or C₄₋₆ cycloalkyl;
R⁶ may be a straight chain or branched chain C₁₋₃ alkoxy; and
n may be an integer from 1 to 3.

In addition, R¹ may be hydrogen, halogen, a straight chain or branched chain C₁₋₂ alkyl unsubstituted or substituted with one or more halogens, or a straight chain or branched chain C₁₋₃ alkoxycarbonyl;
R² may be -NR_{b1}R_{b2},
R_{b1} and R_{b2} may be each independently hydrogen, unsubstituted or substituted C₁₋₂ alkyl, or R_{b1} and R_{b2} may be taken together with the nitrogen to which they are attached to form an unsubstituted or substituted 6-membered heterocycloalkyl, wherein the substituent of the substituted C₁₋₂ alkyl may be -NR_{c1}R_{c2}, and the substituent of the substituted 6-membered heterocycloalkyl may be -NR_{c1}R_{c2} or C₁₋₂ alkyl, and
R_{c1} and R_{c2} may be each independently hydrogen, a straight chain or branched chain C₁₋₃ alkyl, or R_{c1} and R_{c2} may be taken together with the nitrogen to which they are attached to form an unsubstituted or substituted 6-membered heterocycloalkyl, wherein the substituent of the substituted 6-membered heterocycloalkyl may be C₁₋₂ alkyl; and
R³ and R⁵ may be each independently hydrogen or C₁₋₂ alkyl;
R⁴ may be C₁₋₂ alkyl;
R⁶ may be methoxy;
R⁷ and R⁸ may be each independently hydrogen or C₁₋₂ alkyl;
n may be 1 or 2; and
p and q may be each independently an integer from 1 to 3. 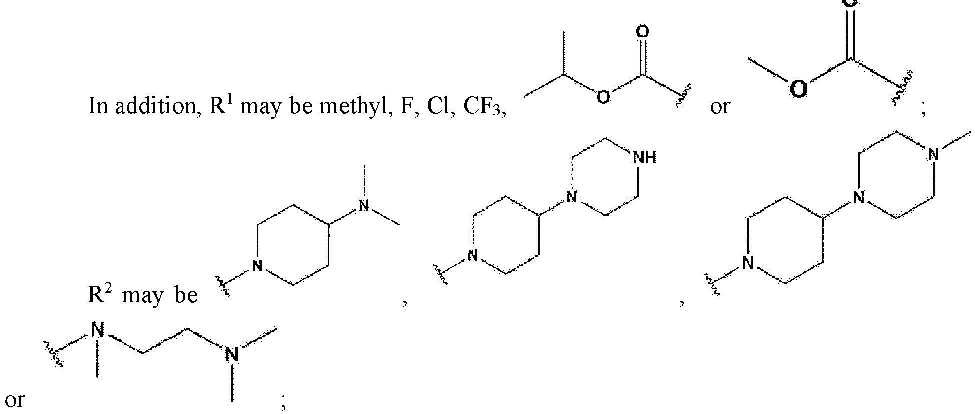
R³ may be hydrogen;
R⁴ may be methyl or ethyl;
R⁵ may be hydrogen;
R⁶ may be methoxy;
R⁷ may be hydrogen;
R⁸ may be hydrogen;
n may be 1 or 2; and
p and q may be each independently an integer from 1 to 3.

The term "heterocycloalkyl", unless otherwise specified, includes a monovalent saturated moiety consisting of 1 to 3 rings comprising 1, 2, 3 or 4 heteroatoms selected from N, O or S. Two or three rings may comprise a bridged, fused or spiro heterocycloalkyl, and may be, for example, pyridine, pyrazine, pyrimidine, pyridazine, piperazine, piperidine, pyrazole, oxazole, thiazole, or morpholine.

Examples of the compound represented by Formula 1 according to the present invention may include the following compounds:
<1> 5-chloro-N2-(4-(4-(dimethylamino)piperidin-1-yl)-2-methoxyphenyl)-N4-(1-(methylsulfonyl)indolin-7-yl)pyrimidine-2,4-diamine;
<2> 5-chloro-N2-(2-methoxy-4-(4-(4-methylpiperazin-1-yl)piperidin-1-yl)phenyl)-N4-(1-(methylsulfonyl)indolin-7-yl)pyrimidine-2,4-diamine;
<3> 5-chloro-N2-(4-((2-(dimethylamino)ethyl)(methyl)amino)-2-methoxyphenyl)-N4-(1-(methylsulfonyl)indolin-7-yl)pyrimidine-2,4-diamine;
<4> 5-chloro-N2-(4-(4-(dimethylamino)piperidin-1-yl)-2-methoxyphenyl)-N4-(1-(methylsulfonyl)-1,2,3,4-tetrahydroquinolin-8-yl)pyrimidine-2,4-diamine;
<5> 5-chloro-N2-(2-methoxy-4-(4-(4-methylpiperazin-1-yl)piperidin-1-yl)phenyl)-N4-(1-(methylsulfonyl)-1,2,3,4-tetrahydroquinolin-8-yl)pyrimidine-2,4-diamine;
<6> 5-chloro-N2-(4-((2-(dimethylamino)ethyl)(methyl)amino)-2-methoxyphenyl)-N4-(1-(methylsulfonyl)-1,2,3,4-tetrahydroquinolin-8-yl)pyrimidine-2,4-diamine;
<7> N2-(2-methoxy-4-(4-(4-methylpiperazin-1-yl)piperidin-1-yl)phenyl)-5-methyl-N4-(1-(methylsulfonyl)indolin-7-yl)pyrimidine-2,4-diamine;
<8> 5-fluoro-N2-(2-methoxy-4-(4-(4-methylpiperazin-1-yl)piperidin-1-yl)phenyl)-N4-(1-(methylsulfonyl)indolin-7-yl)pyrimidine-2,4-diamine;
<9> N2-(2-methoxy-4-(4-(4-methylpiperazin-1-yl)piperidin-1-yl)phenyl)-N4-(1-(methylsulfonyl)indolin-7-yl)-5-(trifluoromethyl)pyrimidine-2,4-diamine;
<10> isopropyl 2-((2-methoxy-4-(4-(4-methylpiperazin-1-yl)piperidin-1-yl)phenyl)amino)-4-((1-(methylsulfonyl)indolin-7-yl)amino)pyrimidine-5-carboxylate;
<11> methyl 2-((2-methoxy-4-(4-(4-methylpiperazin-1-yl)piperidin-1-yl)phenyl)amino)-4-((1-(methylsulfonyl)indolin-7-yl)amino)pyrimidine-5-carboxylate;
<12> 1-(7-((5-chloro-2-((2-methoxy-4-(4-(4-methylpiperazin-1-yl)piperidin-1-yl)phenyl)amino) pyrimidin-4-yl)amino)indolin-1-yl)ethan-1-one;
<13> 5-chloro-N4-(1-(ethylsulfonyl)indolin-7-yl)-N2-(2-methoxy-4-(4-(4-methylpiperazin-1-yl)piperidin-1-yl)phenyl)pyrimidine-2,4-diamine;
<14> 1-(7-((5-chloro-2-((2-methoxy-4-(4-(4-methylpiperazin-1-yl)piperidin-1-yl)phenyl)amino)pyrimidin-4-yl)amino)indolin-1-yl)propan-1-one;
<15> 1-(8-((5-chloro-2-((2-methoxy-4-(4-(4-methylpiperazin-1-yl)piperidin-1-yl)phenyl)amino)pyrimidin-4-yl)amino)-3,4-dihydroquinolin-1(2H)-yl)ethan-1-one;
<16> 1-(8-((5-chloro-2-((2-methoxy-4-(4-(4-methylpiperazin-1-yl)piperidin-1-yl)phenyl)amino)pyrimidin-4-yl)amino)-3,4-dihydroquinolin-1(2H)-yl)propan-1-one; and
<17> 5-chloro-N2-(2-methoxy-4-(4-(piperazin-1-yl)piperidin-1-yl)phenyl)-N4-(1-(methylsulfonyl)indolin-7-yl)pyrimidine-2,4-diamine.

The compound represented by Formula 1 of the present invention may be used in the form of a pharmaceutically acceptable salt, and an acid addition salt formed by a pharmaceutically acceptable free acid is useful as the salt. Acid addition salts are obtained from inorganic acids such as hydrochloric acid, nitric acid, phosphoric acid, sulfuric acid, hydrobromic acid, hydroiodic acid, nitrous acid, phosphorous acid, and the like, non-toxic organic acids such as aliphatic mono- and dicarboxylates, phenyl-substituted alkanoate, hydroxy alkanoate and alkanedioate, aromatic acids, aliphatic and aromatic sulfonic acids, and organic acids such as trifluoroacetic acid, acetate, benzoic acid, citric acid, lactic acid, maleic acid, gluconic acid, methanesulfonic acid, 4-toluenesulfonic acid, tartaric acid, fumaric acid, and the like. These pharmaceutically non-toxic salts include sulfate, pyrosulfate, bisulfate, sulfite, bisulfite, nitrate, phosphate, monohydrogen phosphate, dihydrogen phosphate, metaphosphate, pyrophosphate chloride, bromide, iodide, fluoride, acetate, propionate, decanoate, caprylate, acrylate, formate, isobutyrate, caprate, heptanoate, propiolate, oxalate, malonate, succinate, suberate, sebacate, fumarate, maleate, butyne-1,4-dioate, hexane-1,6-dioate, benzoate, chlorobenzoate, methylbenzoate, dinitrobenzoate, hydroxybenzoate, methoxybenzoate, phthalate, terephthalate, benzenesulfonate, toluenesulfonate, chlorobenzenesulfonate, xylenesulfonate, phenylacetate, phenylpropionate, phenylbutyrate, citrate, lactate, β-hydroxybutyrate, glycolate, malate, tartrate, methanesulfonate, propanesulfonate, naphthalene-1-sulfonate, naphthalene-2-sulfonate, mandelate, and the like.

The acid addition salt according to the present invention may be prepared by conventional methods. The acid addition salt according to the present invention may be prepared, for example, by dissolving the derivative of Formula 1 in an organic solvent such as methanol, ethanol, acetone, methylene chloride, acetonitrile, and the like, adding an organic acid or an inorganic acid, filtering and drying the resulting precipitate, or it may be prepared by distilling the solvent and excess acid under reduced pressure, then drying it, and crystallizing it in an organic solvent.

In addition, a pharmaceutically acceptable metal salt may be prepared using a base. The alkali metal or alkaline earth metal salt is obtained, for example, by dissolving the compound in an excess of alkali metal hydroxide or alkaline earth metal hydroxide solution, filtering the undissolved compound salt, and evaporating and drying the filtrate. In this case, it is pharmaceutically appropriate to prepare a sodium, potassium, or calcium salt as a metal salt. In addition, the corresponding salt is obtained by reacting an alkali metal or alkaline earth metal salt with a suitable negative salt (e.g., silver nitrate).

Furthermore, the present invention includes not only the compound represented by Formula 1 and a pharmaceutically acceptable salt thereof, but also a solvate, a stereoisomer, a hydrate, and the like that may be prepared therefrom.

The term "hydrate" refers to a compound of the present invention or a salt thereof containing a stoichiometric or non-stoichiometric amount of water bound by non-covalent intermolecular forces. The hydrate of the compound represented by Formula 1 of the present invention may contain a stoichiometric or non-stoichiometric amount of water bound by non-covalent intermolecular forces. The hydrate may contain more than 1 equivalent of water, preferably 1 to 5 equivalents of water. The hydrate may be prepared by crystallizing the compound represented by Formula 1 of the present invention, a stereoisomer thereof, or a pharmaceutically acceptable salt thereof from water or a water-containing solvent.

The term "solvate" refers to the compound of the present invention or a salt thereof containing a stoichiometric or non-stoichiometric amount of solvent bound by non-covalent intermolecular forces. Preferred solvents therefor include solvents that are volatile, non-toxic, and/or suitable for administration to humans.

The term "isomer" refers to the compound of the present invention or a salt thereof that has the same chemical formula or molecular formula but is structurally or sterically different. These isomers include structural isomers such as tautomers, stereoisomers such as R or S isomers with asymmetric carbon centers, geometric isomers (trans, cis), and optical isomers (enantiomers). All these isomers and mixtures thereof are also included within the scope of the present invention.

In another aspect of the present invention, there is provided a pharmaceutical composition for preventing or treating cancer, containing the compound represented by Formula 1, a stereoisomer thereof, a solvate thereof, a hydrate thereof, or a pharmaceutically acceptable salt thereof as an active ingredient.

In this case, the cancer is at least one selected from the group consisting of pseudomyxoma, intrahepatic biliary tract cancer, hepatoblastoma, liver cancer, thyroid cancer, colon cancer, testicular cancer, myelodysplastic syndrome, glioblastoma, oral cancer, lip cancer, mycosis fungoides, acute myeloid leukemia, acute lymphocytic leukemia, basal cell cancer, ovarian epithelial cancer, ovarian germ cell cancer, breast cancer, brain cancer, pituitary adenoma, multiple myeloma, gallbladder cancer, biliary tract cancer, large intestine cancer, chronic myeloid leukemia, chronic lymphocytic leukemia, retinoblastoma, choroidal melanoma, ampulla of Vater cancer, bladder cancer, peritoneal cancer, parathyroid cancer, adrenal cancer, sinonasal cancer, non-small cell lung cancer, tongue cancer, astrocytoma, small cell lung cancer, pediatric brain cancer, pediatric lymphoma, pediatric leukemia, small intestine cancer, meningioma, esophageal cancer, glioma, renal pelvis cancer, kidney cancer, heart cancer, duodenum cancer, malignant soft tissue cancer, malignant bone cancer, malignant lymphoma, malignant mesothelioma, malignant melanoma, eye cancer, vulvar cancer, ureteral cancer, urethral cancer, cancer of unknown primary site, gastric lymphoma, stomach cancer, gastric carcinoid, gastrointestinal stromal cancer, Wilms cancer, breast cancer, sarcoma, penile cancer, pharyngeal cancer, gestational trophoblastic disease, cervical cancer, endometrial cancer, uterine sarcoma, prostate cancer, metastatic bone cancer, metastatic brain cancer, mediastinal cancer, rectal cancer, rectal carcinoid, vaginal cancer, spinal cord cancer, acoustic neuroma, pancreatic cancer, salivary gland cancer, Kaposi's sarcoma, Paget's disease, tonsil cancer, squamous cell carcinoma, lung adenocarcinoma, lung cancer, squamous cell carcinoma of the lung, skin cancer, anal cancer, rhabdomyosarcoma, laryngeal cancer, pleura cancer, blood cancer, and thymic cancer, and the cancer may be a cancer expressing a mutation in at least one selected from the group consisting of EGFR, HER2, ALK, FAK, FLT3, JAK3, KIT, and PLK4.

In addition, the compound, a stereoisomer thereof, a solvate thereof, a hydrate thereof, or a pharmaceutically acceptable salt thereof may inhibit EGFR (epidermal growth factor receptor) mutation, and wherein the EGFR mutation may be at least one selected from the group consisting of EGFR del19, EGFR T790M, EGFR C797S, EGFR L858R and EGFR Ex20 insertion mutation, and preferably it may be at least one selected from the group consisting of EGFR del19, EGFR del19/T790M, EGFR del19/T790M/C797S, EGFR L858R, EGFR L858R/T790M, EGFR L858R/T790M/C797S, EGFR A763_Y764insFHEA, EGFR V769_D770insASV and EGFR D770_N771insSVD, etc.

In addition, the compound, a stereoisomer thereof, a solvate thereof, a hydrate thereof, or a pharmaceutically acceptable salt thereof may inhibit HER2 mutation, and wherein the HER2 mutation may be at least one selected from the group consisting of HER2 A775_G776insYVMA, HER2 G776_delinsVC, etc.

In addition, a pharmaceutical composition for preventing or treating cancer, containing the compound represented by Formula 1, a stereoisomer thereof, or a pharmaceutically acceptable salt thereof as an active ingredient may be administered as an individual therapeutic agent or in combination with other anticancer agents in use.

In addition, a pharmaceutical composition for preventing or treating cancer, containing the compound represented by Formula 1, a stereoisomer thereof or a pharmaceutically acceptable salt thereof as an active ingredient may enhance the anticancer effect by administering it in combination with an anticancer agent.

The compound represented by Formula 1 of the present invention exhibits a relatively weak EGFR activity inhibitory effect on wild type EGFR, while exhibiting a high selective inhibitory effect on EGFR and HER2 mutations, and especially a high inhibitory ability against a triple mutation of EGFR del19/T790M/C797S or EGFR L858R/T790M/C797S, and EGFR Ex20 insertion mutation, and/or HER2 mutation.

It may be seen that the compound represented by Formula 1 of the present invention, when administered alone, has a lower cell survival rate against the Ba/F3 Del19/T790M/C797S cell line with an EGFR triple mutation than the conventional drug. In addition, the compound represented by Formula 1 of the present invention has a significantly reduced cell survival rate when administered in combination with the conventional drug compared to when administered alone. Therefore, it may be seen that the compound represented by Formula 1 of the present invention not only has an excellent cancer cell killing ability against cell lines with an EGFR triple mutation, an EGFR Ex20 insertion mutation, and a HER2 mutation when administered alone, but also has a significantly increased anticancer effect when administered in combination with the conventional drug.

Therefore, the compound represented by Formula 1 according to the present invention exhibits a higher inhibitory ability against EGFR and HER2 mutations than wild type EGFR, and in particular, exhibits a significantly superior inhibitory effect against an EGFR mutation of EGFR L858R/T790M/C797S, and may be advantageously used in the treatment of cancer expressing EGFR mutations of EGFR del19, EGFR del19/T790M, EGFR del19/T790M/C797S, EGFR L858R, EGFR L858R/T790MS, EGFR L858R/T790M/C797S, EGFR A763_Y764insFHEA, EGFR V769_D770insASV and EGFR D770_N771insSVD, etc. In particular, the compound represented by Formula 1 according to the present invention has a significantly excellent inhibitory ability against the triple mutation EGFR del19/T790M/C797S or EGFR L858R/T790M/C797S, and thus may also be advantageously used in the treatment of cancer expressing EGFR del19/T790M/C797S or EGFR L858R/T790M/C797S, EGFR A763_Y764insFHEA, EGFR V769_D770insASV and EGFR D770_N771insSVD.

In addition, when administered in combination with the conventional drug, the compound represented by Formula 1 according to the present invention exhibits a synergistic effect, and thus may be advantageously used in combination with the conventional drug.

The compound represented by Formula 1 or a pharmaceutically acceptable salt thereof may be administered in various oral and parenteral dosage forms during clinical administration. When formulated, it is prepared using diluents or excipients such as commonly used fillers, extenders, binders, wetting agents, disintegrants, and surfactants. Solid preparations for oral administration include tablets, pills, powders, granules, capsules, and the like. These solid preparations are prepared by mixing one or more compounds with at least one or more excipients, such as starch, calcium carbonate, sucrose or lactose, gelatin, etc. In addition to simple excipients, lubricants such as magnesium stearate and talc are also used. Liquid preparations for oral administration include suspensions, internal solutions, emulsions, and syrups. In addition to the commonly used simple diluents such as water and liquid paraffin, various excipients such as wetting agents, sweeteners, fragrances, and preservatives may be included. Preparations for parenteral administration include sterilized aqueous solutions, non-aqueous solvents, suspensions, and emulsions. Non-aqueous solvents and suspensions may include propylene glycol, polyethylene glycol, vegetable oil such as olive oil, and injectable esters such as ethyl oleate.

A pharmaceutical composition containing the compound represented by Formula 1 or a pharmaceutically acceptable salt thereof as an active ingredient may be administered parenterally, and parenteral administration is by subcutaneous injection, intravenous injection, intramuscular injection, or intrathoracic injection.

In this case, in order to formulate a formulation for parenteral administration, the compound represented by Formula 1 or a pharmaceutically acceptable salt thereof is mixed with water along with a stabilizer or buffer to prepare a solution or suspension, which may be prepared in an ampoule or vial unit dosage form. The composition may be sterilized and/or may contain auxiliaries such as preservatives, stabilizers, wetting agents or emulsification accelerators, salts and/or buffers for adjusting osmotic pressure, and other therapeutically useful substances, and may be formulated according to conventional methods such as mixing, granulating or coating.

Formulations for oral administration include, for example, tablets, pills, hard/soft capsules, solutions, suspensions, emulsions, syrups, granules, elixirs, troches, and the like. In addition to an active ingredient, these formulations contain diluents (e.g. lactose, dextrose, sucrose, mannitol, sorbitol, cellulose and/or glycine), lubricants (e.g. silica, talc, stearic acid and its magnesium or calcium salts and/or or polyethylene glycol). The tablets may contain binders such as magnesium aluminum silicate, starch paste, gelatin, methylcellulose, sodium carboxymethylcellulose and/or polyvinylpyrrolidine, and in some cases, may contain disintegrants or effervescent mixtures such as starch, agar, alginic acid or its sodium salt, and/or absorbents, colorants, flavoring agents, and sweeteners.

In another aspect of the present invention, there is provided a health functional food for preventing or improving cancer, containing the compound represented by Formula 1, a stereoisomer thereof, a solvate thereof, a hydrate thereof, or a pharmaceutically acceptable salt thereof as an active ingredient.

The cancer may be at least one selected from the group consisting of pseudomyxoma, intrahepatic biliary tract cancer, hepatoblastoma, liver cancer, thyroid cancer, colon cancer, testicular cancer, myelodysplastic syndrome, glioblastoma, oral cancer, lip cancer, mycosis fungoides, acute myeloid leukemia, acute lymphocytic leukemia, basal cell cancer, ovarian epithelial cancer, ovarian germ cell cancer, breast cancer, brain cancer, pituitary adenoma, multiple myeloma, gallbladder cancer, biliary tract cancer, large intestine cancer, chronic myeloid leukemia, chronic lymphocytic leukemia, retinoblastoma, choroidal melanoma, ampulla of Vater cancer, bladder cancer, peritoneal cancer, parathyroid cancer, adrenal cancer, sinonasal cancer, non-small cell lung cancer, tongue cancer, astrocytoma, small cell lung cancer, pediatric brain cancer, pediatric lymphoma, pediatric leukemia, small intestine cancer, meningioma, esophageal cancer, glioma, renal pelvis cancer, kidney cancer, heart cancer, duodenum cancer, malignant soft tissue cancer, malignant bone cancer, malignant lymphoma, malignant mesothelioma, malignant melanoma, eye cancer, vulvar cancer, ureteral cancer, urethral cancer, cancer of unknown primary site, gastric lymphoma, stomach cancer, gastric carcinoid, gastrointestinal stromal cancer, Wilms cancer, breast cancer, sarcoma, penile cancer, pharyngeal cancer, gestational trophoblastic disease, cervical cancer, endometrial cancer, uterine sarcoma, prostate cancer, metastatic bone cancer, metastatic brain cancer, mediastinal cancer, rectal cancer, rectal carcinoid, vaginal cancer, spinal cord cancer, acoustic neuroma, pancreatic cancer, salivary gland cancer, Kaposi's sarcoma, Paget's disease, tonsil cancer, squamous cell carcinoma, lung adenocarcinoma, lung cancer, squamous cell carcinoma of the lung, skin cancer, anal cancer, rhabdomyosarcoma, laryngeal cancer, pleura cancer, and thymic cancer, and the cancer may be a cancer expressing mutations in EGFR and HER2.

The compound represented by Formula 1 according to the present invention exhibits a high inhibitory ability against EGFR and HER2 mutations, and thus may be added to health functional foods such as foods and beverages as a health functional food composition for preventing or improving cancer.

The compound represented by Formula 1 according to the present invention may be added as is to food or used together with other foods or food ingredients, and may be used appropriately according to conventional methods. The mixing amount of the active ingredient may be appropriately determined depending on the purpose of use (prevention or improvement). In general, the amount of the above compound in health food may be added in an amount of 0.1 to 90 parts by weight of the total weight of the food. However, in the case of long-term intake for the purpose of health and hygiene or health control, the above amount may be below the above range, and since there is no problem in terms of safety, the active ingredient may be used in an amount above the above range.

In addition, the health functional beverage composition of the present invention has no particular restrictions on other ingredients other than containing the above compound as an active ingredient in the indicated proportion, and may contain various flavoring agents or natural carbohydrates as additional ingredients like ordinary beverages. Examples of the above-mentioned natural carbohydrates include monosaccharides such as glucose, fructose, etc.; disaccharides such as maltose, sucrose, etc.; and polysaccharides, such as common sugars such as dextrin and cyclodextrin, and sugar alcohols such as xylitol, sorbitol, and erythritol. In addition to those mentioned above, natural flavoring agents (thaumatin, stevia extract (e.g., rebaudioside A, glycyrrhizin, etc.)) and synthetic flavoring agents (saccharin, aspartame, etc.) may be advantageously used as flavoring agents. The proportion of natural carbohydrates is generally about 1 to 20 g, preferably about 5 to 12 g, per 100 g of the composition of the present invention.

Furthermore, in addition to the above, the compound represented by Formula 1 according to the present invention may contain various nutrients, vitamins, minerals (electrolytes), flavoring agents such as synthetic and natural flavoring agents, colorants and enhancing agents (cheese, chocolate, etc.), pectic acid and its salts, alginic acid and its salts, organic acids, protective colloidal thickeners, pH adjusters, stabilizers, preservatives, glycerin, alcohol, carbonating agents used in carbonated beverages, and the like. In addition, the compound represented by Formula 1 of the present invention may contain pulp for the production of natural fruit juice, fruit juice beverages, and vegetable beverages.

In another aspect of the present invention, there is provided a method for preventing or treating cancer, comprising: administering a pharmaceutical composition or a health functional food containing the compound represented by Formula 1, a stereoisomer thereof, a solvate thereof, a hydrate thereof, or a pharmaceutically acceptable salt thereof as an active ingredient to a subject in need thereof.

In another aspect of the present invention, there is provided a use of a pharmaceutical composition or a health functional food containing the compound represented by Formula 1, a stereoisomer thereof, a solvate thereof, a hydrate thereof, or a pharmaceutically acceptable salt thereof for the prevention or treatment of cancer.

The pharmaceutical composition of the present invention is administered in a "pharmaceutically effective amount." As used herein, the term "pharmaceutically effective amount" refers to an amount sufficient to treat a disease with a reasonable benefit/risk ratio applicable to medical treatment or improvement, and the effective dose level may be determined based on factors including the type and severity of the subject, age, gender, activity of the drug, sensitivity to the drug, time of administration, route of administration and excretion rate, duration of treatment, concurrently used drugs, and other factors well known in the medical field. For example, an effective amount of 0.001 mg/kg to 1000 mg/kg, 0.01 mg/kg to 100 mg/kg, or 0.1 to 20 mg/kg, or 0.1 to 500 mg/kg is included. The upper quantitative limit of the pharmaceutical composition of the present invention may be selected and implemented by one of ordinary skill in the art within an appropriate range.

### Mode for Carrying out the Invention

Hereinafter, the present invention will be described in detail by way of examples and experimental examples.

However, the following examples and experimental examples are merely illustrative of the present invention, and the content of the present invention is not limited to the following examples and experimental examples.

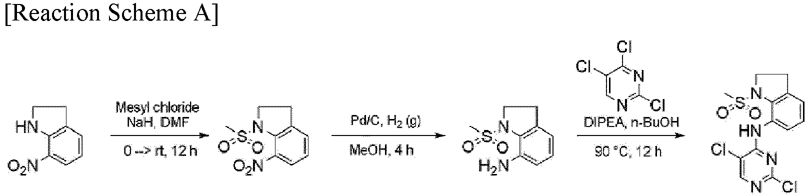

According to Reaction Scheme A above, the compounds of Preparation Examples 1 to 3 were obtained.

### <Preparation Example 1> Preparation of 1-(methylsulfonyl)-7-nitroindoline

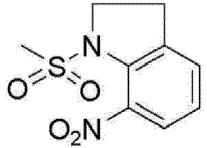

To a stirred solution of 7-nitroindoline (2.0 g, 1.21 mmol) in DMF were added NaH (0.8 g, 3.6 mmol) and mesyl chloride (4.7 ml, 6.0 mmol) at 0°C. The resulting mixture was heated to room temperature and stirred for 12 hours. The reaction mixture was quenched with cold water. The precipitated solid was filtered, washed with water, and dried to obtain 1-(methylsulfonyl)-7-nitroindoline (87%) as a pure yellow solid.

¹H NMR (500 MHz, Chloroform-*d*) δ 7.79 (d, *J* = 8.3 Hz, 1 H), 7.57 - 7.48 (m, 1 H), 7.22 (t, *J* = 7.8 Hz, 1 H), 4.29 (t, *J* = 7.8 Hz, 2 H), 3.26 (s, 3 H), 3.22 (t, *J* = 7.8 Hz, 2 H); LCMS: 243.0 [M+H⁺].

### <Preparation Example 2> Preparation of 1-(methylsulfonyl)indolin-7-amine

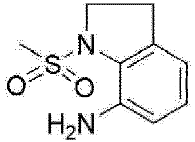

To a stirred solution of 1-(methylsulfonyl)-7-nitroindoline (2.2 g, 9.0 mmol) in methanol was added 10% Pd/C (0.1 g, 0.9 mmol). The resulting mixture was stirred under 1 atm hydrogen gas for 4 hours at room temperature. The reaction mixture was filtered through Celite and concentrated under reduced pressure to obtain 1-(methylsulfonyl)indolin-7-amine (90%) as a brown solid.

¹H NMR (400 MHz, Chloroform-*d*) δ 7.00 (td, *J* = 7.8, 2.3 Hz, 1 H), 6.70 (d, *J* = 7.5 Hz, 1 H), 6.61 (dd, *J* = 8.1, 2.3 Hz, 1 H), 4.60 (s, 2 H), 4.11 (td, *J* = 7.8, 2.3 Hz, 2 H), 3.03 (td, *J* = 7.6, 2.3 Hz, 2 H), 2.87 (s, 3H); LCMS: 213.0 [M+H⁺].

### <Preparation Example 3> Preparation of N-(2,5-dichloropyrimidin-4-yl)-1-(methylsulfonyl)indolin-7-amine

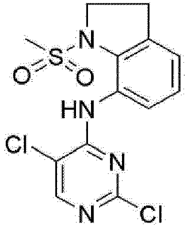

To a stirred solution of 1-(methylsulfonyl)indolin-7-amine (500 mg, 2.35 mmol) in n-BuOH (5 ml) were added diisopropylethylamine (1.3 ml, 7.05 mmol) and 2,4,5-trichloropyrimidine (518 mg, 2.82 mmol) at room temperature. The reaction mixture was heated to 90°C for 14 hours. The reaction mixture was cooled to room temperature and concentrated under reduced pressure. The resulting material was filtered and washed with n-BuOH to obtain N-(2,5-dichloropyrimidin-4-yl)-1-(methylsulfonyl)indolin-7-amine as an off-white solid.

¹H NMR (400 MHz, Chloroform-*d*) δ 9.35 (s, 1 H), 8.21 (s, 1 H), 8.01 (d, *J* = 8.4 Hz, 1 H), 7.42 - 7.26 (m, 2 H), 7.18 (d, *J* = 7.5 Hz, 1 H), 4.16 (td, *J* = 7.5, 2.2 Hz, 2 H), 3.15 (t, *J* = 7.7 Hz, 2 H), 2.92 (s, 3 H); LCMS: 359.8 [M+H⁺].

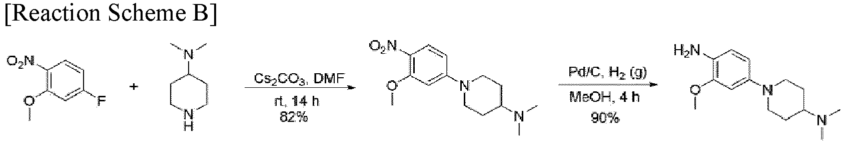

According to Reaction Scheme B above, the compounds of Preparation Examples 4 and 5 were obtained.

### <Preparation Example 4> Preparation of (3-methoxy-4-nitrophenyl)-N,N-dimethylpiperidin-4-amine

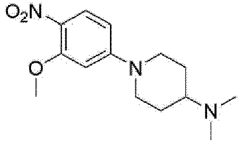

To a stirred solution of 4-fluoro-2-methoxy-1-nitrobenzene (10 g, 58.4 mmol) in DMF (150 ml) were added N,N-dimethylpiperidin-4-amine (7.5 g, 58.4 mmol) and cesium carbonate (3.8 g, 116.8 mol) at room temperature. The resulting mixture was stirred at room temperature overnight. The reaction mixture was quenched with cold water and stirred for 15 minutes. The precipitated solid was filtered and concentrated under reduced pressure to obtain 1-(3-methoxy-4-nitrophenyl)-N,N-dimethylpiperidin-4-amine (14.0 g, 85%) as a yellow solid.

¹H NMR (500 MHz, Chloroform-*d*) δ 8.01 (d, *J* = 9.4 Hz, 1 H), 6.44 (dd, *J* = 9.4, 2.6 Hz, 1 H), 6.33 (d, *J* = 2.5 Hz, 1 H), 3.97 (s, 3 H), 3.97-3.86 (m, 2 H), 3.03 - 2.94 (m, 2 H), 2.50 - 2.36 (m, 1 H), 2.33 (s, 6 H), 2.04-1.89 (m, 2 H), 1.68 - 1.51 (m, 2 H); LCMS: 279.0 [M+H⁺].

### <Preparation Example 5> Preparation of 1-(4-amino-3-methoxyphenyl)-N,N-dimethylpiperidin-4-amine

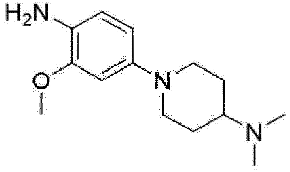

To a stirred solution of 1-(3-methoxy-4-nitrophenyl)-N,N-dimethylpiperidin-4-amine (14.0 g, 50.1 mmol) in methanol (150 ml) was added 10% Pd/C (1.0 g, 10.0 mmol). The resulting mixture was stirred under 1 atm hydrogen gas at room temperature for 4 hours. The reaction mixture was filtered through Celite and concentrated under reduced pressure to obtain 1-(4-amino-3-methoxyphenyl)-N,N-dimethylpiperidin-4-amine (11.2 g, 90%) as a brown solid.

¹H NMR (500 MHz, Chloroform-*d*) δ 6.65 (d, *J* = 8.3 Hz, 1 H), 6.55 (d, *J* = 2.4 Hz, 1 H), 6.45 (dd, *J* = 8.4, 2.5 Hz, 1 H), 3.86 (s, 3 H), 3.57 - 3.50 (m, 4 H), 2.67 - 2.60 (m, 2H), 2.34 (s, 6H), 2.89-2.19 (m, 1 H), 1.96 - 1.90 (m, 2 H); LCMS: 249.0 [M+H⁺].

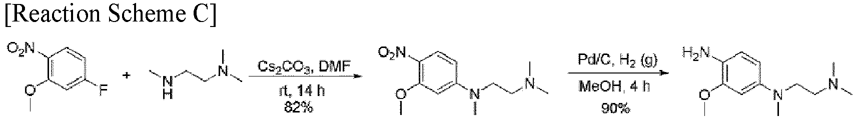

According to Reaction Scheme C above, the compounds of Preparation Examples 6 and 7 were obtained.

### <Preparation Example 6> Preparation of N1-(3-methoxy-4-nitrophenyl)-N1,N2,N2-trimethylethane-1,2-diamine

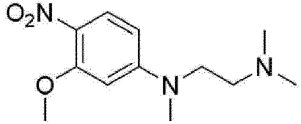

The same method as in Preparation Example 4 was performed to obtain N1-(3-methoxy-4-nitrophenyl)-N1,N2,N2-trimethylethane-1,2-diamine (84%).

¹H NMR (500 MHz, Chloroform-*d*) δ 8.03 (d, *J* = 9.3 Hz, 1 H), 6.25 (dd, *J* = 9.4, 2.6 Hz, 1 H), 6.13 (d, *J* = 2.6 Hz, 1 H), 3.96 (s, 3 H), 3.59 - 3.53 (m, 2 H), 3.11 (s, 3 H), 2.53 (t, *J* = 7.3 Hz, 2 H), 2.32 (s, 6 H); LCMS: 253.0 [M+H⁺].

### <Preparation Example 7> Preparation of N1-(2-(dimethylamino)ethyl)-3-methoxy-N1-methylbenzene-1,4-diamine

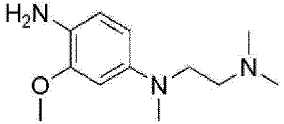

The same method as in Preparation Example 5 was performed to obtain N1-(2-(dimethylamino)ethyl)-3-methoxy-N1-methylbenzene-1,4-diamine (96%).

¹H NMR (400 MHz, Methanol-*d*₄) δ 7.23 (d, *J* = 8.7 Hz, 1 H), 6.61 (d, *J* = 2.6 Hz, 1 H), 6.52 (dd, *J* = 8.8, 2.7 Hz, 1 H), 4.02 (s, 3 H), 3.84 (t, *J* = 7.3 Hz, 2 H), 3.43 - 3.35 (m, 5 H), 3.06 (s, 3 H), 2.98 (s, 6 H); LCMS: 223.0 [M+H⁺].

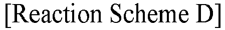

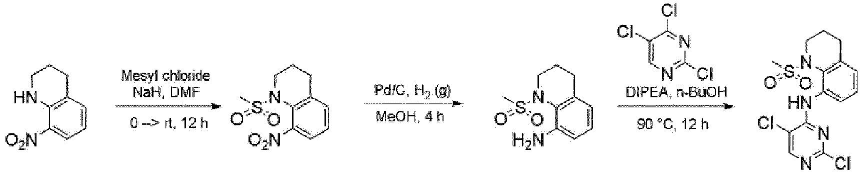

According to Reaction Scheme D above, the compounds of Preparation Examples 8 to 10 were obtained.

### <Preparation Example 8> Preparation of 1-(methylsulfonyl)-8-nitro-1,2,3,4-tetrahydroquinoline

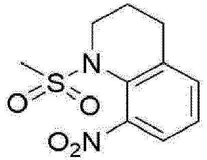

The same method as in Preparation Example 1 was performed to obtain 1-(methylsulfonyl)-8-nitro-1,2,3,4-tetrahydroquinoline (69%).

¹H NMR (400 MHz, Chloroform-*d*) δ 7.80 - 7.73 (m, 1 H), 7.40 (d, *J* = 7.8 Hz, 1 H), 7.25 (dq, *J* = 7.4, 3.5, 2.5 Hz, 1 H), 3.72 (s, 2 H), 3.14 (s, 3 H), 2.95 (td, *J* = 7.3, 2.2 Hz, 2 H), 2.23 (s, 2 H), 1.43 - 1.34 (m, 2 H); LCMS: 257.0 [M+H⁺].

### <Preparation Example 9> Preparation of 1-(methylsulfonyl)-1,2,3,4-tetrahydroquinolin-8-amine

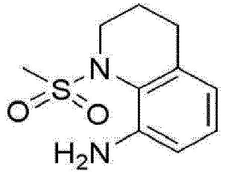

The same method as in Preparation Example 2 was performed to obtain 1-(methylsulfonyl)-1,2,3,4-tetrahydroquinolin-8-amine (92%).

¹H NMR (400 MHz, Chloroform-*d*) δ 7.03 (td, *J* = 7.8, 2.2 Hz, 1 H), 6.66 (d, *J* = 8.1 Hz, 1 H), 6.59 (d, *J* = 7.5 Hz, 1 H), 4.44 (s, 2 H), 3.73 (s, 2 H), 2.94 (s, 3 H), 2.83 - 2.71 (m, 2 H), 2.17 - 2.00 (m, 2 H); LCMS: 227.0 [M+H⁺].

### <Preparation Example 10> Preparation of N-(2,5-dichloropyrimidin-4-yl)-1-(methylsulfonyl)-1,2,3,4-tetrahydroquinolin-8-amine

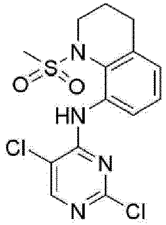

The same method as in Preparation Example 3 was performed to obtain N-(2,5-dichloropyrimidin-4-yl)-1-(methylsulfonyl)-1,2,3,4-tetrahydroquinolin-8-amine (77%).

¹H NMR (400 MHz, Chloroform-*d*) δ 9.05 (s, 1 H), 8.21 (s, 1 H), 7.93 (d, *J* = 8.3 Hz, 1 H), 7.41 - 7.29 (m, 1 H), 7.09 (d, *J* = 7.7 Hz, 1 H), 3.89 - 3.59 (m, 2 H), 2.97 (s, 3 H), 2.89 (t, *J* = 7.5 Hz, 2 H), 2.18 (d, *J* = 9.8 Hz, 2 H); LCMS: 374.0 [M+H⁺].

According to the following reaction scheme, the compound of Preparation Example 11 was obtained.

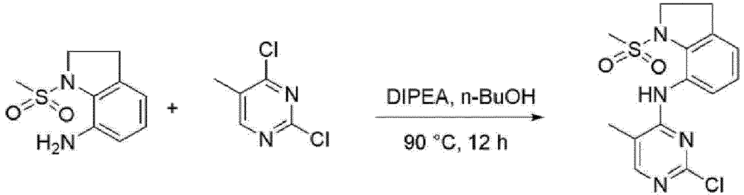

### <Preparation Example 11> Preparation of N-(2-chloro-5-methylpyrimidin-4-yl)-1-(methylsulfonyl)indolin-7-amine

The same method as in Preparation Example 3 was performed to obtain N-(2-chloro-5-methylpyrimidin-4-yl)-1-(methylsulfonyl)indolin-7-amine (29%).

¹H NMR (500 MHz, Chloroform-*d*) δ 8.86 (s, 1 H), 8.16 (dd, *J* = 8.4, 1.1 Hz, 1 H), 8.02 (s, 1 H), 7.35 - 7.27 (m, 1 H), 7.13 (dq, *J* = 7.4, 1.1 Hz, 1 H), 4.17 (t, *J* = 7.5 Hz, 2 H), 3.13 (t, *J* = 7.5 Hz, 2 H), 2.89 (s, 3 H), 2.22 (d, *J=* 0.9 Hz, 3 H); LCMS: 338.8 [M+H⁺].

According to the following reaction scheme, the compound of Preparation Example 12 was obtained.

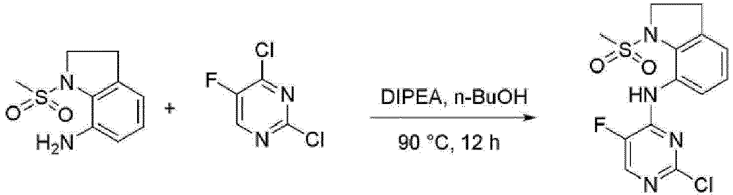

### <Preparation Example 12> Preparation of N-(2-chloro-5-fluoropyrimidin-4-yl)-1-(methylsulfonyl)indolin-7-amine

The same method as in Preparation Example 3 was performed to obtain N-(2-chloro-5-fluoropyrimidin-4-yl)-1-(methylsulfonyl)indolin-7-amine (82%).

¹H NMR (400 MHz, Chloroform-*d*) δ 9.28 (s, 1 H), 8.14 (bs, 2 H), 7.31 (d, *J* = 12.3 Hz, 1 H), 4.18 (t, *J* = 7.7 Hz, 2 H), 3.15 (t, *J* = 7.8 Hz, 2 H), 2.91 (s, 3 H); LCMS: 342.0 [M+H⁺].

According to the following reaction scheme, the compound of Preparation Example 13 was obtained.

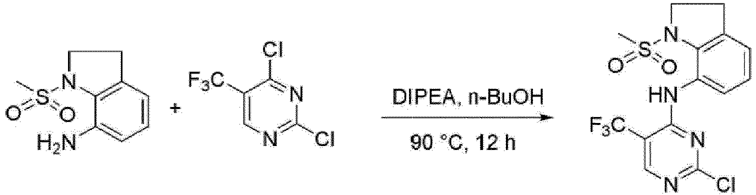

### <Preparation Example 13> Preparation of N-(2-chloro-5-(trifluoromethyl)pyrimidin-4-yl)-1-(methylsulfonyl)indolin-7-amine

The same method as in Preparation Example 3 was performed to obtain N-(2-chloro-5-(trifluoromethyl)pyrimidin-4-yl)-1-(methylsulfonyl)indolin-7-amine (24%), which was separated using HPLC.

¹H NMR (400 MHz, Chloroform-*d*) δ 9.20 (s, 1 H), 8.44 (cs, 1 H), 7.80 (dd, *J* = 8.2, 1.1 Hz, 1 H), 7.35 - 7.29 (m, 1 H), 7.22 (dq, *J* = 7.4, 1.1 Hz, 1 H), 4.13 (t, *J* = 7.5 Hz, 2 H), 3.17 (t, *J* = 7.4 Hz, 2 H), 2.94 (s, 3 H); LCMS: 394.0 [M+H²⁺].

According to the following reaction scheme, the compound of Preparation Example 14 was obtained.

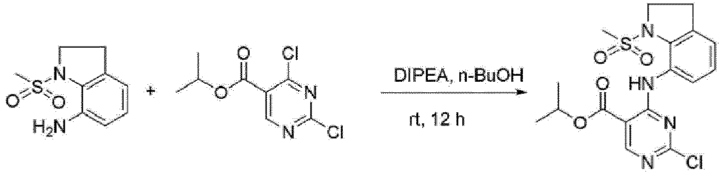

### <Preparation Example 14> Preparation of isopropyl 2-chloro-4-((1-(methylsulfonyl)indolin-7-yl)amino)pyrimidine-5-carboxylate

The same method as in Preparation Example 3 was performed to obtain isopropyl 2-chloro-4-((1-(methylsulfonyl)indolin-7-yl)amino)pyrimidine-5-carboxylate (84%).

¹H NMR (500 MHz, Chloroform-*d*) δ 10.71 (s, 1 H), 8.83 (s, 1 H), 7.94 (dd, *J* = 8.3, 1.0 Hz, 1 H), 7.31 - 7.27 (m, 2 H), 7.18 (dq, *J* = 7.4, 1.1 Hz, 1 H), 5.41 - 5.29 (m, 1 H), 4.13 (t, *J* = 7.4 Hz, 2 H), 3.20 - 3.15 (m, 2 H), 3.00 (s, 3 H), 1.41 (d, *J* = 6.3 Hz, 6 H); LCMS: 411.8 [M+H⁺].

According to the following reaction scheme, the compound of Preparation Example 15 was obtained.

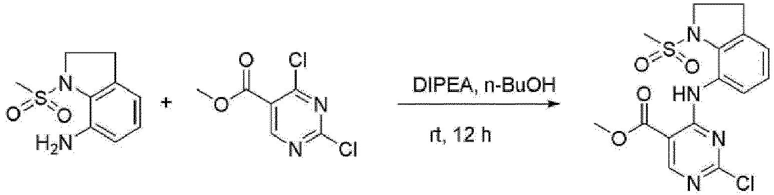

### <Preparation Example 15> Preparation of methyl 2-chloro-4-((1-(methylsulfonyl)indolin-7-yl)amino)pyrimidine-5-carboxylate

The same method as in Preparation Example 3 was performed to obtain methyl 2-chloro-4-((1-(methylsulfonyl)indolin-7-yl)amino)pyrimidine-5-carboxylate (88%).

¹H NMR (500 MHz, DMSO-*d*₆) δ 10.44 (s, 1H), 8.80 (s, 1H), 7.72 (dd, *J* = 7.3, 2.1 Hz, 1H), 7.31 - 7.24 (m, 2H), 4.05 (t, *J* = 7.4 Hz, 2H), 3.90 (s, 3H), 3.12 (t, *J* = 7.4 Hz, 2H), 3.07 (s, 3H); LCMS: 383.1 [M+H⁺].

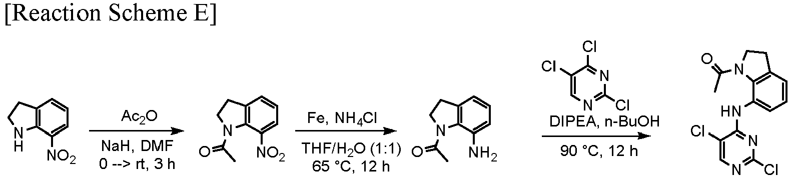

According to Reaction Scheme E above, the compounds of Preparation Examples 16 to 18 were obtained.

### <Preparation Example 16> Preparation of 1-(7-nitroindolin-1-yl)ethan-1-one

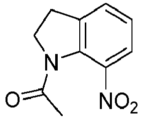

The same method as in Preparation Example 1 was performed to obtain 1-(7-nitroindolin-1 -yl)ethan-1 -one.

¹H NMR (300 MHz, Chloroform-d) δ 7.64 (dd, J = 8.2, 1.1 Hz, 1 H), 7.46 - 7.39 (m, 1 H), 7.15 (t, J = 7.8 Hz, 1 H), 4.25 (t, J = 8.1 Hz, 2 H), 3.23 (t, J = 8.0 Hz, 2 H), 2.27 (s 3 H). LCMS: 207.2 [M+H⁺].

### <Preparation Example 17> Preparation of 1-(7-aminoindolin-1-yl)-ethan-1-one

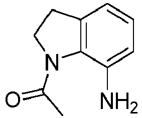

To a stirred solution of 1-(methylsulfonyl)-7-nitroindoline (1.0 g, 4.84 mmol) in 20 mL of THF/H₂O (1:1) were added iron powder (1.3 g, 24.24 mmol) and NH₄Cl (1.3 g 24.24 mmol) at room temperature to obtain 1-(7-aminoindolin-1-yl)-ethan-1-one (90%) as a brown solid.

¹H NMR (400 MHz, Chloroform-d) δ 6.97 (t, J = 7.6 Hz, 1 H), 6.66 (dt, J = 7.3, 1.1 Hz, 1 H), 6.60 (dd, J = 8.1, 1.0 Hz, 1 H), 4.82 (s, 2 H), 4.07 (t, J = 7.8 Hz, 2 H), 3.07 (t, J = 7.7 Hz, 2 H), 2.33 (s, 3 H). LCMS: 177.8 [M+H⁺].

### <Preparation Example 18> Preparation of 1-(7-((2,5-dichloropyrimidin-4-yl)amino)indolin-1-yl)ethan-1-one

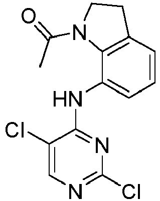

The same method as in Preparation Example 3 for 1-(7-aminoindolin-1-yl)-ethan-1-one (1.0 g, 5.67 mmol) was performed to obtain 1-(7-((2,5-dichloropyrimidin-4-yl)amino)indolin-1-yl)ethan-1-one (80%) as a dark green solid.

¹H NMR (400 MHz, DMSO-d6) δ 10.47 (s, 1 H), 8.38 (s, 1 H), 7.64 - 7.57 (m, 1 H), 7.22 (t, J = 7.7 Hz, 1 H), 7.15 (dd, J = 7.3, 1.3 Hz, 1 H), 4.16 (t, J = 7.8 Hz, 2 H), 3.11 (t, J = 7.7 Hz, 2 H), 2.32 (s, 3 H).. LCMS: 324.8 [M+H⁺].

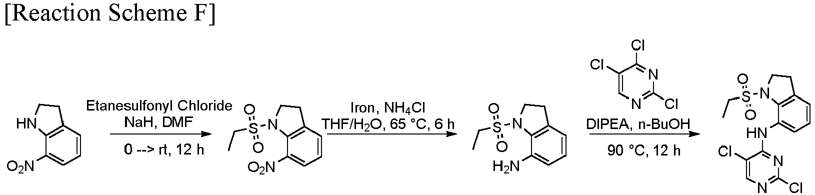

According to Reaction Scheme F above, the compounds of Preparation Examples 19 to 21 were obtained.

### <Preparation Example 19> Preparation of 1-(ethylsulfonyl)-7-nitroindoline

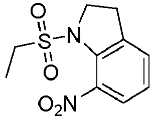

The same method as in Preparation Example 1 was performed to obtain 1-(ethylsulfonyl)-7-nitroindoline (59%).

¹H NMR (400 MHz, Chloroform-*d*) δ 7.78 (dd, *J* = 8.3, 1.1 Hz, 1 H), 7.49 (dd, *J* = 7.4, 1.2 Hz, 1 H), 7.21 (dd, *J* = 8.2, 7.4 Hz, 1 H), 4.28 (t, *J* = 7.7 Hz, 2 H), 3.42 (q, *J* = 7.4 Hz, 2 H), 3.21 (tt, *J* = 7.7, 1.0 Hz, 2 H), 1.52 (t, *J* = 7.4 Hz, 3 H). LCMS: 257.0 [M+H⁺].

### <Preparation Example 20> Preparation of 1-(ethylsulfonyl)indolin-7-amine

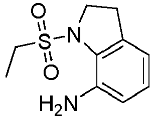

The same method as in Preparation Example 17 was performed to obtain 1-(ethylsulfonyl)indolin-7-amine (92%).

¹H NMR (400 MHz, Chloroform-*d*) δ 7.01 - 6.89 (m, 1 H), 6.69 (t, *J* = 8.2 Hz, 1 H), 6.60 (t, *J* = 8.7 Hz, 1 H), 4.61 (s, 2 H), 4.20 - 3.93 (m, 2 H), 3.16 - 2.76 (m, 4 H), 1.50 - 1.20 (m, 3 H). LCMS: 227.0 [M+H⁺].

### <Preparation Example 21> Preparation of N-(2,5-dichloropyrimidin-4-yl)-1-(ethylsulfonyl)indolin-7-amine

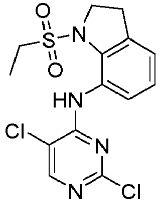

The same method as in Preparation Example 3 was performed to obtain N-(2,5-dichloropyrimidin-4-yl)-1-(ethylsulfonyl)indolin-7-amine (71%).

¹H NMR (500 MHz, Chloroform-*d*) δ 9.33 (s, 1 H), 8.21 (s, 1 H), 7.92 (d, *J* = 8.2 Hz, 1 H), 7.33 - 7.24 (m, 2 H), 7.16 (d, *J* = 7.4 Hz, 1 H), 4.13 (t, *J* = 7.4 Hz, 2 H), 3.16 (t, *J* = 7.5 Hz, 2 H), 3.11 (q, *J=* 7.4 Hz, 2 H), 1.50 - 1.40 (m, 3 H). LCMS: 374.0 [M+2H⁺].

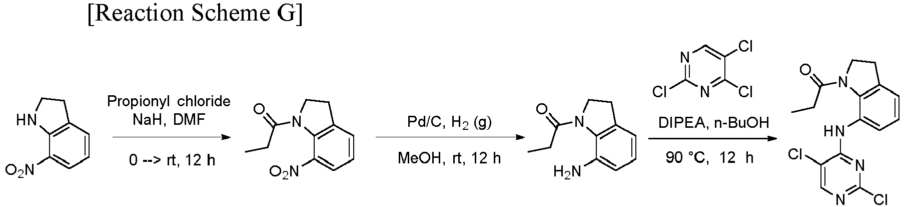

According to Reaction Scheme G above, the compounds of Preparation Examples 22 to 24 were obtained.

### Preparation Example 22> Preparation of 1-(7-nitroindolin-1-yl)propan-1-one

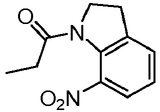

The same method as in Preparation Example 1 was performed to obtain 1-(7-nitroindolin-1-yl)propan-1-one.

¹H NMR (500 MHz, DMSO-d6) δ 7.66 - 7.56 (m, 2 H), 7.23 (t, J = 7.8 Hz, 1 H), 4.24 (t, J = 8.1 Hz, 2 H), 3.21 (t, J = 8.2 Hz, 2 H), 2.54 (dd, J = 8.7, 6.2 Hz, 2 H), 1.05 (td, J = 7.4, 2.5 Hz, 3 H). LCMS: 220.0 [M+H⁺].

### <Preparation Example 23> Preparation of 1-(7-aminoindolin-1-yl)propan-1-one

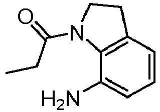

The same method as in Preparation Example 2 was performed to obtain 1-(7-aminoindolin-1 -yl)propan-1 -one.

¹H NMR (500 MHz, DMSO-d6) δ 6.86 (t, J = 7.6 Hz, 1 H), 6.61 - 6.50 (m, 7.6 Hz, 2 H), 5.42 (s, 2 H), 4.03 (t, J = 7.8 Hz, 2 H), 2.96 (t, J = 7.9 Hz, 2 H), 2.56 (q, J = 7.5 Hz, 2 H), 1.10 (t, J = 7.4 Hz, 3 H). LCMS: 190.2 [M+H⁺].

### <Preparation Example 24> Preparation of 1-(7-((2,5-dichloropyrimidin-4-yl)amino)indolin-1-yl)propan-1-one

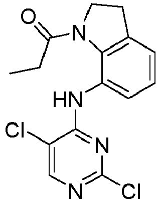

The same method as in Preparation Example 3 was performed to obtain 1-(7-((2,5-dichloropyrimidin-4-yl)amino)indolin-1-yl)propan-1-one.

¹H NMR (500 MHz, DMSO-d6) δ 10.35 (s, 1 H), 8.38 (d, J = 2.4 Hz, 1 H), 7.62 (d, J = 8.2 Hz, 1 H), 7.22 (d, J = 7.8 Hz, 1 H), 7.17 (d, J = 7.5 Hz, 1 H), 4.16 (s, 2 H), 3.12 (s, 2 H), 2.64 (d, J = 7.4 Hz, 2 H), 1.11 (t, J = 7.4 Hz, 3 H). LCMS: 337.8 [M+H⁺].

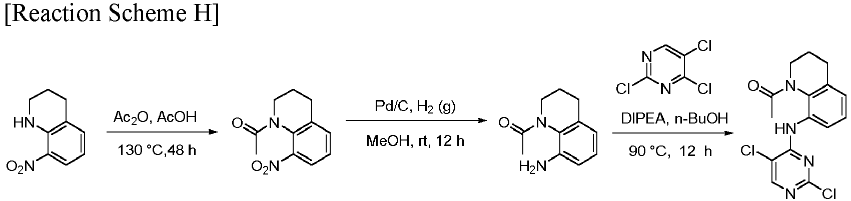

According to Reaction Scheme H above, the compounds of Preparation Examples 25 to 27 were obtained.

### <Preparation Example 25> Preparation of 1-(8-nitro-3,4-dihydroquinolin-1(2H)-yl)ethan-1-one

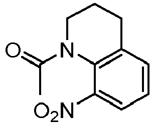

The same method as in Preparation Example 16 was performed using 8-nitro-3,4-dihydroquinoline to obtain 1-(8-nitro-3,4-dihydroquinolin-1(2H)-yl)ethan-1-one.

¹H NMR (500 MHz, DMSO-d6) δ 7.76 - 7.65 (m, 1 H), 7.53 (d, J = 7.6 Hz, 1 H), 7.30 (t, J = 7.9 Hz, 1 H), 3.82 (s, 2 H), 2.83 (s, 2 H), 2.19 (d, J = 2.2 Hz, 3 H), 1.98 (s, 2 H). LCMS: 220.4 [M+H⁺].

### <Preparation Example 26> Preparation of 1-(8-amino-3,4-dihydroquinolin-1(2H)-yl)ethan-1-one

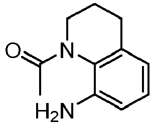

The same method as in Preparation Example 2 was performed to obtain 1-(8-amino-3,4-dihydroquinolin-1(2H)-yl)ethan-1-one.

¹H NMR (500 MHz, DMSO-d6) δ 6.89 (t, J = 7.6 Hz, 1 H), 6.61 (d, J = 7.9 Hz, 1 H), 6.44 (d, J = 7.3 Hz, 1 H), 5.12 (s, 2 H), 4.63 - 4.48 (m, 2 H), 2.68 - 2.59 (m, 1H), 2.58 - 2.53 (m, 1 H), 2.33 - 2.26 (m, 1 H), 2.15 - 2.04 (m, 1H), 1.90 (s, 3 H), 1.61 - 1.49 (m, 1 H). LCMS: 190.2 [M+H⁺].

### <Preparation Example 27> Preparation of 1-(8-((2,5-dichloropyrimidin-4-yl)amino)-3,4-dihydroquinolin-1(2H)-yl)ethan-1-one

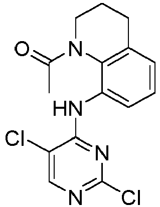

The same method as in Preparation Example 3 was performed to obtain 1-(8-((2,5-dichloropyrimidin-4-yl)amino)-3,4-dihydroquinolin-1 (2H)-yl)ethan-1-one.

¹H NMR (400 MHz, DMSO-d6) δ 8.72 (s, 1 H), 8.37 (s, 1 H), 7.52 - 7.08 (m, 3 H), 4.31 - 3.98 (m, 2 H), 3.12 - 2.69 (m, 2 H), 2.20 (d, J = 49.1 Hz, 3 H), 1.91 - 1.53 (m, 2 H). LCMS: 337.8 [M+H⁺].

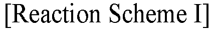

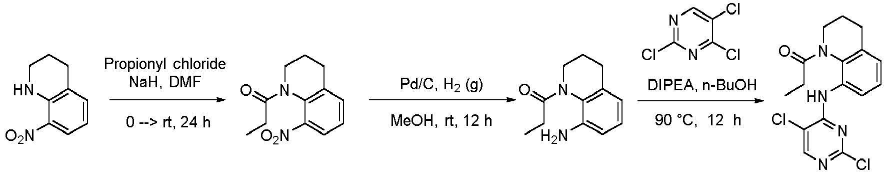

According to Reaction Scheme I above, the compounds of Preparation Examples 28 to 30 were obtained.

### <Preparation Example 28> Preparation of 1-(8-nitro-3,4-dihydroquinolin-1(2H)-yl)propan-1-one

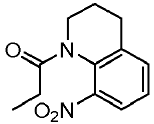

The same method as in Preparation Example 1 was performed to obtain 1-(8-nitro-3,4-dihydroquinolin-1(2H)-yl)propan-1 -one.

¹H NMR (500 MHz, DMSO-d6) δ 7.70 (d, J = 8.1 Hz, 1 H), 7.53 (d, J = 7.6 Hz, 1H), 7.29 (t, J = 8.0 Hz, 1 H), 3.83 (bs, 2 H), 2.82 (s, 2 H), 2.54 (s, 2 H), 2.12 - 1.79 (m, 2 H), 1.01 (t, J = 7.5 Hz, 3 H). LCMS: 234.4 [M+H⁺].

### <Preparation Example 29> Preparation of 1-(8-amino-3,4-dihydroquinolin-1(2H)-yl)propan-1-one

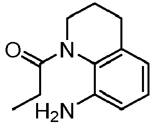

The same method as in Preparation Example 2 was performed to obtain 1-(8-amino-3,4-dihydroquinolin-1(2H)-yl)propan-1 -one.

¹H NMR (500 MHz, DMSO-d6) δ 6.89 (t, J = 7.7 Hz, 1 H), 6.60 (d, J = 8.1 Hz, 1 H), 6.44 (d, J = 7.5 Hz, 1 H), 5.10 (s, 2 H), 4.68 - 4.55 (m, 2 H), 2.70 - 2.59 (m, 1 H), 2.54 (d, J = 4.9 Hz, 1H), 2.41 (dq, J = 15.4, 7.6 Hz, 1 H), 2.24 (q, J = 12.2, 11.0 Hz, 1 H), 2.10 (s, 1 H), 2.02 (dt, J = 15.9, 7.6 Hz, 1 H), 1.53 (s, 1 H), 1.09 (t, J = 7.5 Hz, 1 H), 0.90 (t, J = 7.5 Hz, 3 H). LCMS: 204.4 [M+H⁺].

### <Preparation Example 30> Preparation of 1-(8-((2,5-dichloropyrimidin-4-yl)amino)-3,4-dihydroquinolin-1(2H)-yl)propan-1-one

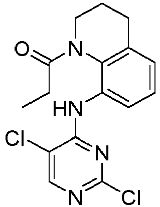

The same method as in Preparation Example 3 was performed to obtain 1-(8-((2,5-dichloropyrimidin-4-yl)amino)-3,4-dihydroquinolin-1(2H)-yl)propan-1-one.

¹H NMR (400 MHz, DMSO-d6) δ 8.69 (s, 1 H), 8.36 (s, 1 H), 7.44 (s, 1 H), 7.33 - 7.09 (m, 2 H), 4.17 (d, J = 78.3 Hz, 2 H), 3.28 (s, 1 H), 2.83 (s, 1 H), 2.74 (s, 1 H), 2.58 (s, 1 H), 2.33 - 1.52 (m, 2H), 1.05 (s, 2 H), 0.83 (s, 1 H). LCMS: 351.8 [M+H⁺].

According to the following reaction scheme, the compound of Example 1 was obtained.

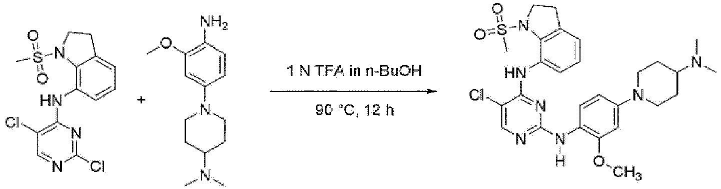

### <Example 1> Preparation of 5-chloro-N2-(4-(4-(dimethylamino)piperidin-1-yl)-2-methoxyphenyl)-N4-(1-(methylsulfonyl)indolin-7-yl)pyrimidine-2,4-diamine

To a stirred solution of N-(2,5-dichloropyrimidin-4-yl)-1-(methylsulfonyl)indolin-7-amine (100 mg, 0.27 mmol) in 1 N TFA in n-BuOH (3 ml) was added 1-(4-amino-3-methoxyphenyl)-N,N-dimethylpiperidin-4-amine (81.3 mg, 0.27 mmol) at room temperature. The resulting mixture was heated to 90°C for 14 hours. The reaction mixture was diluted with DCM. The organic layer was washed with a saturated NaHCO₃ aqueous solution, followed by water and brine, dried over MgSO₄, and then evaporated under reduced pressure. The resulting material was purified using column chromatography (5-10% methanol in DCM as an eluent) to obtain 5-chloro-N2-(4-(4-(dimethylamino)piperidin-1-yl)-2-methoxyphenyl)-N4-(1-(methylsulfonyl)indolin-7-yl)pyrimidine-2,4-diamine (49%) as a white solid.

¹H NMR (400 MHz, Chloroform-*d*) δ 8.95 (s, 1 H), 8.14 - 8.03 (m, 2 H), 8.04 - 7.98 (m, 1 H), 7.36 - 7.24 (m, 3 H), 7.15 (dd, *J* = 7.4, 1.2 Hz, 1 H), 6.54 (d, *J* = 2.6 Hz, 1 H), 6.43 (dd, *J* = 8.8, 2.6 Hz, 1 H), 4.17 (t, *J* = 7.5 Hz, 2 H), 3.87 (s, 3 H), 3.66 (d, *J* = 11.9 Hz, 2 H), 3.16 (t, *J* = 7.5 Hz, 2 H), 2.94 (s, 3 H), 2.72 (td, *J* = 12.3, 2.4 Hz, 2 H), 2.64 - 2.40 (m, 6 H), 2.08 (d, *J* = 12.3 Hz, 2 H), 1.79 (qd, *J=* 12.1, 4.1 Hz, 2 H); LCMS: 572.8 [M+H⁺].

According to the following reaction scheme, the compound of Example 2 was obtained.

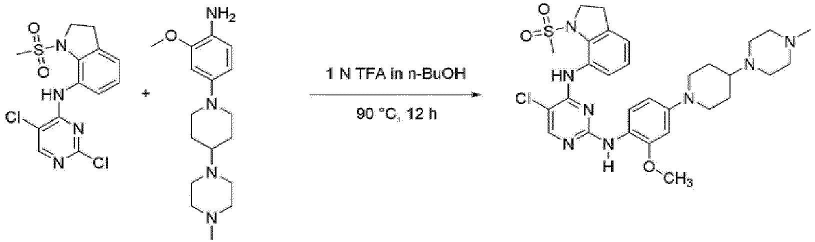

### <Example 2> Preparation of 5-chloro-N2-(2-methoxy-4-(4-(4-methylpiperazin-1-yl)piperidin-1-yl)phenyl)-N4-(1-(methylsulfonyl)indolin-7-yl)pyrimidine-2,4-diamine

The same method as in Example 1 was performed to obtain 5-chloro-N2-(2-methoxy-4-(4-(4-methylpiperazin-1-yl)piperidin-1-yl)phenyl)-N4-(1-(methylsulfonyl)indolin-7-yl)pyrimidine-2,4-diamine (54%).

¹H NMR (400 MHz, Chloroform-*d*) δ 8.94 (s, 1 H), 8.10 - 7.99 (m, 3 H), 7.33 - 7.23 (m, 3 H), 7.17 - 7.12 (m, 1 H), 6.54 (d, *J* = 2.5 Hz, 1 H), 6.42 (dd, *J* = 8.8, 2.5 Hz, 1 H), 4.17 (t, *J* = 7.5 Hz, 2 H), 3.87 (s, 3 H), 3.65 (d, *J* = 12.1 Hz, 2 H), 3.15 (t, *J* = 7.5 Hz, 2 H), 2.94 (s, 3 H), 2.87 - 2.57 (m, 9 H), 2.47 (m, 1 H), 2.41 (s, 3 H), 1.99 (d, *J=* 12.1 Hz, 2 H), 1.82 - 1.67(m, 2 H); LCMS: 627.9 [M+H⁺].

According to the following reaction scheme, the compound of Example 3 was obtained.

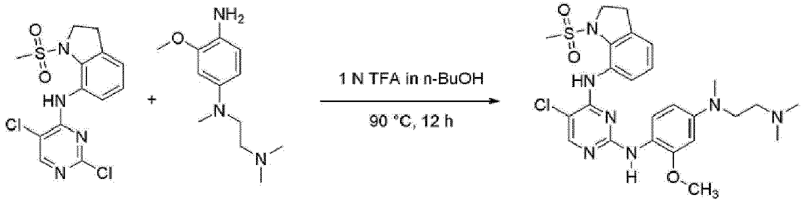

### <Example 3> Preparation of 5-chloro-N2-(4-((2-(dimethylamino)ethyl)(methyl)amino)-2-methoxyphenyl)-N4-(1-(methylsulfonyl)indolin-7-yl)pyrimidine-2,4-diamine

The same method as in Example 1 was performed to obtain 5-chloro-N2-(4-((2-(dimethylamino)ethyl)(methyl)amino)-2-methoxyphenyl)-N4-(1-(methylsulfonyl)indolin-7-yl)pyrimidine-2,4-diamine (43%).

¹H NMR (400 MHz, Chloroform-*d*) δ 8.93 (s, 1 H), 8.08 - 8.02 (m, 2 H), 7.97 (d, *J* = 8.9 Hz, 1H), 7.28 - 7.24 (m, 1 H), 7.18 (s, 1 H), 7.15 - 7.10 (m, 1 H), 6.39 (d, *J* = 2.6 Hz, 1 H), 6.26 (dd, *J* = 8.9, 2.7 Hz, 1 H), 4.17 (t, *J* = 7.5 Hz, 2 H), 3.88 (s, 3 H), 3.53 (t, *J* = 7.5 Hz, 2 H), 3.15 (t, *J* = 7.4 Hz, 2 H), 2.96 (s, 3 H), 2.94 (s, 3 H), 2.68 - 2.60 (m, 2 H), 2.44 (s, 6 H); LCMS: 546.8 [M+H⁺].

According to the following reaction scheme, the compound of Example 4 was obtained.

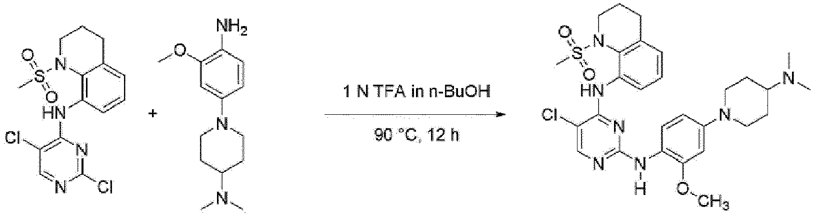

### <Example 4> Preparation of 5-chloro-N2-(4-(4-(dimethylamino)piperidin-1-yl)-2-methoxyphenyl)-N4-(1-(methylsulfonyl)-1,2,3,4-tetrahydroquinolin-8-yl)pyrimidine-2,4-diamine

The same method as in Example 1 was performed to obtain 5-chloro-N2-(4-(4-(dimethylamino)piperidin-1-yl)-2-methoxyphenyl)-N4-(1-(methylsulfonyl)-1,2,3,4-tetrahydroquinolin-8-yl)pyrimidine-2,4-diamine (55%).

¹H NMR (400 MHz, Chloroform-*d*) δ 8.47 (s, 1 H), 8.06 (s, 1 H), 7.99 (d, *J* = 8.9 Hz, 1 H), 7.93 (dd, *J* = 8.2, 1.4 Hz, 1 H), 7.33 - 7.28 (m, 3 H), 7.10 - 7.05 (m, 1 H), 6.54 (d, *J* = 2.6 Hz, 1 H), 6.39 (dd, *J* = 8.9, 2.6 Hz, 1 H), 3.86 (s, 3 H), 3.76 - 3.60 (m, 4 H), 3.02 (s, 3 H), 2.89 (t, *J* = 7.2 Hz, 2 H), 2.71 (td, *J* = 12.2, 2.4 Hz, 2 H), 2.50 - 2.37 (m, 6 H), 2.24 - 2.11 (m, 2 H), 2.03 (d, *J* = 12.5 Hz, 2 H), 1.75 (qd, *J* = 12.1, 3.9 Hz, 2 H); LCMS: 586.9 [M+H⁺].

According to the following reaction scheme, the compound of Example 5 was obtained.

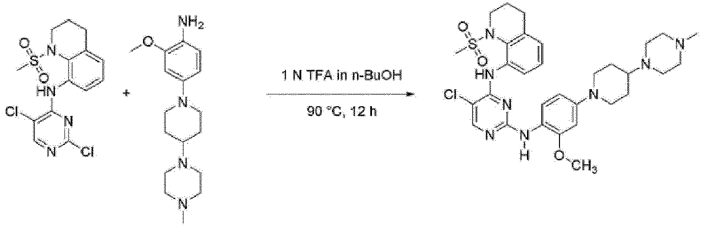

### <Example 5> Preparation of 5-chloro-N2-(2-methoxy-4-(4-(4-methylpiperazin-1-yl)phenyl)-N4-(1-(methylsulfonyl)-1,2,3,4-tetrahydroquinolin-8-yl)pyrimidine-2,4-diamine

The same method as in Example 1 was performed to obtain 5-chloro-N2-(2-methoxy-4-(4-(4-methylpiperazin-1-yl)phenyl)-N4-(1-(methylsulfonyl)-1,2,3,4-tetrahydroquinolin-8-yl)pyrimidine-2,4-diamine (49%).

¹H NMR (400 MHz, Chloroform-*d*) δ 8.46 (s, 1H), 8.05 (s, 1 H), 7.97 (d, *J* = 8.8 Hz, 1 H), 7.93 (dd, *J* = 8.2, 1.4 Hz, 1 H), 7.34 - 7.29 (m, 2 H), 7.08 (dd, *J* = 7.4, 1.4 Hz, 1 H), 6.53 (d, *J* = 2.6 Hz, 1 H), 6.38 (dd, *J* = 8.9, 2.5 Hz, 1 H), 3.86 (s, 3 H), 3.75 - 3.60 (m, 4 H), 3.02 (s, 3 H), 2.89 (t, *J* = 7.2 Hz, 2 H), 2.84 - 2.52 (m, 11 H), 2.39 (s, 3 H), 2.23 - 2.11 (m, 2 H), 1.99 (d, *J*= 12.4 Hz, 2 H), 1.74 (dd, *J=* 12.0, 3.9 Hz, 2 H); LCMS: 641.0 [M+H⁺].

According to the following reaction scheme, the compound of Example 6 was obtained.

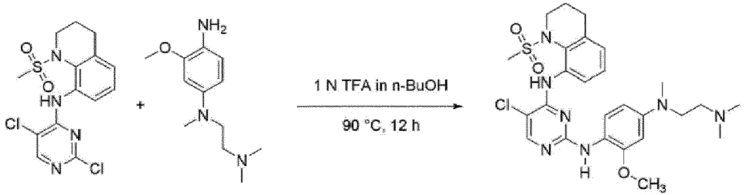

### <Example 6> Preparation of 5-chloro-N2-(4-((2-(dimethylamino)ethyl)(methyl)amino)-2-methoxyphenyl)-N4-(1-(methylsulfonyl)-1,2,3,4-tetrahydroquinolin-8-yl)pyrimidine-2,4-diamine

The same method as in Example 1 was performed to obtain 5-chloro-N2-(4-((2-(dimethylamino)ethyl)(methyl)amino)-2-methoxyphenyl)-N4-(1-(methylsulfonyl)-1,2,3,4-tetrahydroquinolin-8-yl)pyrimidine-2,4-diamine (34%).

¹H NMR (400 MHz, Chloroform-*d*) δ 8.45 (s, 1H), 8.05 (s, 1 H), 7.97 (dd, *J =* 8.2, 1.4 Hz, 1 H), 7.92 (d, *J* = 8.9 Hz, 1 H), 7.31 (d, *J* = 7.8 Hz, 1 H), 7.19 (s, 1 H), 7.06 (dd, *J* = 7.5, 1.4 Hz, 1 H), 6.40 (d, *J* = 2.6 Hz, 1 H), 6.23 (dd, *J* = 8.9, 2.6 Hz, 1 H), 3.88 (s, 3 H), 3.77 - 3.64 (bs, 2 H), 3.62 - 3.51 (bs, 2 H), 3.02 (s, 3 H), 2.96 (s, 3 H), 2.89 (t, *J* = 7.1 Hz, 2 H), 2.69 (s, 2 H), 2.48 (s, 6 H), 2.23 - 2.12 (m, 2 H); LCMS: 561.0 [M+H⁺].

According to the following reaction scheme, the compound of Example 7 was obtained.

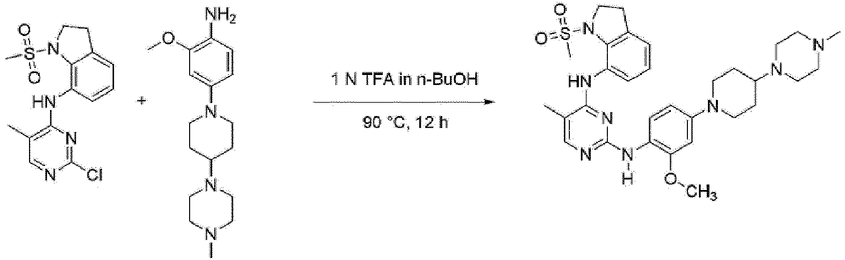

### <Example 7> Preparation of N2-(2-methoxy-4-(4-(4-methylpiperazin-1-yl)piperidin-1-yl)phenyl)-5-methyl-N4-(1-(methylsulfonyl)indolin-7-yl)pyrimidine-2,4-diamine

The same method as in Example 1 was performed to obtain N2-(2-methoxy-4-(4-(4-methylpiperazin-1-yl)piperidin-1-yl)phenyl)-5-methyl-N4-(1-(methylsulfonyl)indolin-7-yl)pyrimidine-2,4-diamine (54%).

¹H NMR (400 MHz, Methanol-*d*₄) δ 7.76 (d, *J* = 8.0 Hz, 1 H), 7.67 (s, 1 H), 7.40 (d, *J*= 8.8 Hz, 1 H), 7.37 - 7.32 (m, 1 H), 7.29 (t, *J* = 7.7 Hz, 1 H), 6.83 (d, *J* = 2.5 Hz, 1H), 6.62 (dd, *J* = 8.8, 2.5 Hz, 1 H), 4.12 (t, *J* = 7.6 Hz, 2 H), 3.88 (s, 3 H), 3.83 (d, *J* = 2.5 Hz, 2 H) 3.27 - 3.06 (m, 6 H), 2.96 (m, 6 H), 2.88 (s, 3 H), 2.57 - 2.52 (m, 5 H) 2.21 (s, 3 H), 2.14 (d, *J* = 12.6 Hz, 2 H), 1.91 - 1.71 (m, 2 H); LCMS: 606.9 [M+H⁺].

According to the following reaction scheme, the compound of Example 8 was obtained.

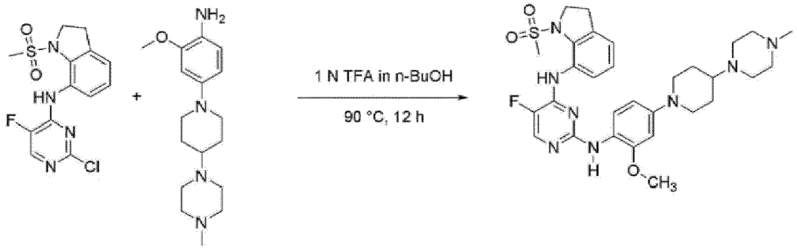

### <Example 8> Preparation of 5-fluoro-N2-(2-methoxy-4-(4-(4-methylpiperazin-1-yl)piperidin-1-yl)-N4-(1-(methylsulfonyl)indolin-7-yl)pyrimidine-2,4-diamine

The same method as in Example 1 was performed to obtain 5-fluoro-N2-(2-methoxy-4-(4-(4-methylpiperazin-1-yl)piperidin-1-yl)-N4-(1-(methylsulfonyl)indolin-7-yl)pyrimidine-2,4-diamine (45%).

¹H NMR (500 MHz, DMSO-*d*₆) δ 8.92 (d, *J* = 2.5 Hz, 1 H), 8.06 (d, *J* = 3.3 Hz, 1 H), 7.98 (d, *J=* 8.0 Hz, 1 H), 7.69 (s, 1 H), 7.56 (d, *J=* 8.7 Hz, 1 H), 7.22 - 7.12 (m, 2 H), 6.61 (d, *J* = 2.5 Hz, 1 H), 6.42 (dd, *J* = 8.8, 2.5 Hz, 1 H), 4.07 (t, *J* = 7.5 Hz, 2 H), 3.78 (s, 3 H), 3.68 (d, *J* = 12.1 Hz, 2 H), 3.12 (t, *J* = 7.5 Hz, 2 H), 3.05 (s, 3 H), 2.64 (td, *J* = 12.2, 2.4 Hz, 2 H), 2.57 - 2.52 (m, 4 H), 2.46 - 2.27 (m, 4 H) 2.20 (s, 3 H), 1.86 (d, *J* = 12.5 Hz, 2 H), 1.52 (dt, *J* = 13.3, 9.6 Hz, 2 H); LCMS: 610.8 [M+H⁺].

According to the following reaction scheme, the compound of Example 9 was obtained.

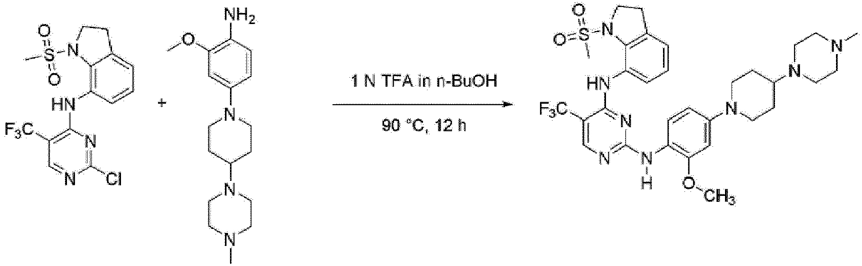

### <Example 9> Preparation of N2-(2-methoxy-4-(4-(4-methylpiperazin-1-yl)piperidin-1-yl)phenyl)-N4-(1-(methylsulfonyl)indolin-7-yl)-5-(trifluoromethyl)pyrimidine-2,4-diamine

The same method as in Example 1 was performed to obtain N2-(2-methoxy-4-(4-(4-methylpiperazin-1-yl)piperidin-1-yl)phenyl)-N4-(1-(methylsulfonyl)indolin-7-yl)-5-(trifluoromethyl)pyrimidine-2,4-diamine (48%).

¹H NMR (400 MHz, Methanol-*d*₄) δ 8.35 (s, 1 H), 7.71 (d, *J* = 8.9 Hz, 1 H), 7.59 (d, *J* = 7.9 Hz, 1 H), 7.37 (dd, *J* = 7.4, 1.4 Hz, 1 H), 7.32 (d, *J* = 7.8 Hz, 1 H), 6.99 (d, *J* = 2.5 Hz, 1 H), 6.72 (dd, *J* = 8.8, 2.5 Hz, 1 H), 4.10 (t, *J* = 7.6 Hz, 2 H), 3.93 (s, 3 H), 3.82 (d, *J* = 12.5 Hz, 2 H), 3.45 - 3.33 (m, 3 H), 3.26 (d, *J=* 12.0 Hz, 3 H), 3.24 - 3.08 (m, 6 H), 2.98 (s, 3 H), 2.92 (s, 3 H), 2.57 - 2.52 (m, 4 H), 2.20 (d, *J=* 13.0 Hz, 2 H), 2.06 - 1.89 (m, 2 H); LCMS: 661.0 [M+H⁺].

According to the following reaction scheme, the compound of Example 10 was obtained.

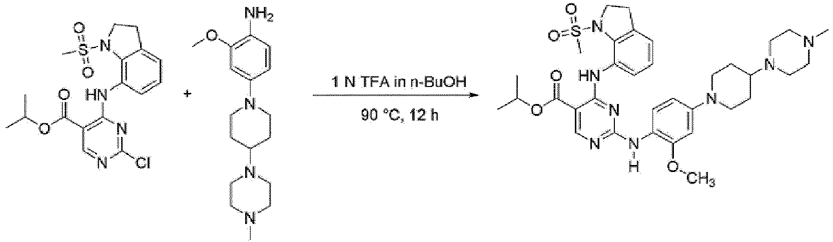

### <Example 10> Preparation of isopropyl 2-((2-methoxy-4-(4-(4-methylpiperazin-1-yl)piperidin-1-yl)phenyl)amino)-4-((1-(methylsulfonyl)indolin-7-yl)amino)pyrimidine-5-carboxylate

The same method as in Example 1 was performed to obtain isopropyl 2-((2-methoxy-4-(4-(4-methylpiperazin-1-yl)piperidin-1-yl)phenyl)amino)-4-((1-(methylsulfonyl)indolin-7-yl)amino)pyrimidine-5-carboxylate (52%).

¹H NMR (400 MHz, DMSO-*d*₆) δ 10.35 (s, 1 H), 8.60 (d, *J* = 9.5 Hz, 2 H), 7.32 (d, *J* = 8.6 Hz, 1 H), 7.16 - 6.93 (m, 2 H), 6.62 (d, *J =* 2.5 Hz, 1 H), 6.43 (dd, *J* = 8.9, 2.5 Hz, 1 H), 5.18 - 5.06 (m, 1 H), 4.00 (t, *J* = 7.1 Hz, 2 H), 3.75 (s, 5 H), 3.12 - 3.01 (m, 5 H), 2.76 - 2.62 (m, 2 H), 2.57 - 2.52 (m, 4 H), 2.42 - 2.23 (m, 5 H), 2.15 (s, 3 H), 1.91 - 1.80 (m, 2 H), 1.58 - 1.45 (m, 2 H), 1.32 (d, *J* = 6.2 Hz, 6 H); LCMS: 679.0 [M+H⁺].

According to the following reaction scheme, the compound of Example 11 was obtained.

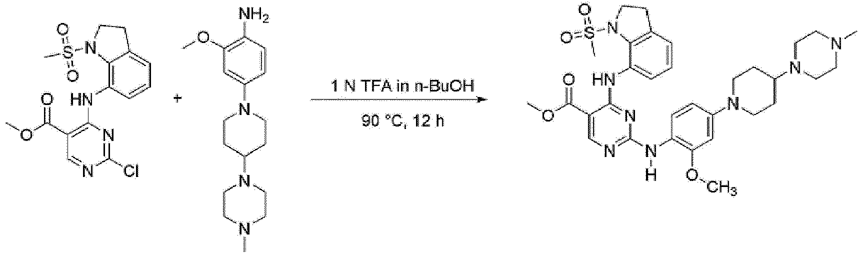

### <Example 11> Preparation of methyl 2-((2-methoxy-4-(4-(4-methylpiperazin-1-yl)piperidin-1-yl)phenyl)amino)-4-((1-(methylsulfonyl)indolin-7-yl)amino)pyrimidine-5-carboxylate

The same method as in Example 1 was performed to obtain methyl 2-((2-methoxy-4-(4-(4-methylpiperazin-1-yl)piperidin-1-yl)phenyl)amino)-4-((1-(methylsulfonyl)indolin-7-yl)amino)pyrimidine-5-carboxylate (44%).

¹H NMR (400 MHz, DMSO-*d*₆) δ 10.32 (s, 1 H), 8.62 (d, *J* = 10.4 Hz, 2 H), 7.87 (s, 1 H), 7.31 (d, *J* = 8.6 Hz, 1 H), 7.12 - 6.93 (m, 2 H), 6.61 (d, *J* = 2.5 Hz, 1 H), 6.50 - 6.38 (m, 1 H), 4.00 (t, *J* = 7.2 Hz, 2 H), 3.82 (s, 3 H), 3.78 - 3.68 (m, 5 H), 3.13 - 3.02 (m, 5 H), 2.69 (t, *J* = 11.7 Hz, 2 H), 2.57 - 2.52 (m, 4 H), 2.41 - 2.24 (m, 5 H), 2.15 (s, 3 H), 1.86 (d, *J* = 12.2 Hz, 2 H), 1.64 - 1.42 (m, 2 H); LCMS: 651.0 [M+H⁺].

According to the following reaction scheme, the compound of Example 12 was obtained.

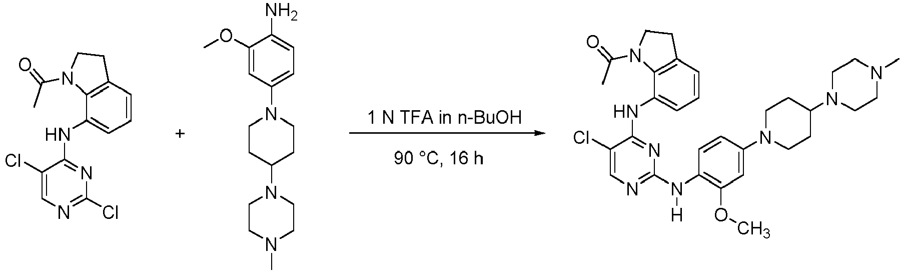

### <Example 12> Preparation of 1-(7-((5-chloro-2-((2-methoxy-4-(4-(4-methylpiperazin-1-yl)piperidin-1-yl)phenyl)amino)pyrimidin-4-yl)amino)indolin-1-yl)ethan-1-one

The same method as in Example 1 was performed to obtain 1-(7-((5-chloro-2-((2-methoxy-4-(4-(4-methylpiperazin-1-yl)piperidin-1-yl)phenyl)amino) pyrimidin-4-yl)amino)indolin-1 -yl)ethan-1 -one.

¹H NMR (400 MHz, DMSO-d6) δ 9.76 (s, 1 H), 8.03 (s, 1 H), 7.74 - 7.63 (m, 2 H), 7.54 (d, J = 8.7 Hz, 1 H), 7.13 (t, J = 7.7 Hz, 1 H), 7.06 (d, J = 7.2 Hz, 1 H), 6.58 (d, J = 2.5 Hz, 1 H), 6.37 (dd, J = 8.8, 2.5 Hz, 1 H), 4.13 (t, J = 7.7 Hz, 2 H), 3.77 (s, 3 H), 3.67 (d, J = 12.2 Hz, 2 H), 3.07 (t, J = 7.7 Hz, 2 H), 2.63 (t, J = 11.9 Hz, 2 H), 2.31 (s, 8 H), 2.14 (s, 3 H), 1.84 (d, J = 12.3 Hz, 2 H), 1.51 (tt, J = 12.7, 6.4 Hz, 2 H).

According to the following reaction scheme, the compound of Example 13 was obtained.

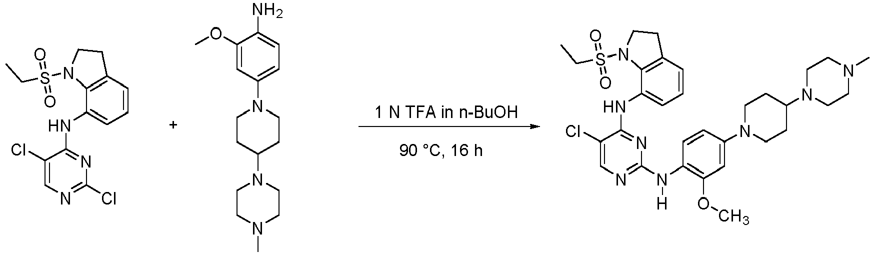

### <Example 13> Preparation of 5-chloro-N4-(1-(ethylsulfonyl)indolin-7-yl)-N2-(2-methoxy-4-(4-(4-methylpiperazin-1-yl)piperidin-1-yl)phenyl)pyrimidine-2,4-diamine

The same method as in Example 1 was performed to obtain 5-chloro-N4-(1-(ethylsulfonyl)indolin-7-yl)-N2-(2-methoxy-4-(4-(4-methylpiperazin-1-yl)piperidin-1-yl)phenyl)pyrimidine-2,4-diamine.

¹H NMR (400 MHz, DMSO-d6) δ 8.88 (s, 1 H), 8.08 (s, 1 H), 7.86 (s, 1 H), 7.82 (t, J = 4.7 Hz, 1 H), 7.44 (d, J = 8.7 Hz, 1 H), 7.19 - 7.10 (m, 2 H), 6.59 (d, J = 2.4 Hz, 1 H), 6.38 (dd, J = 8.7, 2.4 Hz, 1 H), 4.03 (t, J = 7.4 Hz, 2 H), 3.76 (s, 3 H), 3.68 (d, J = 12.3 Hz, 2 H), 3.27 (t, J = 7.3 Hz, 2 H), 3.09 (t, J = 7.4 Hz, 2 H), 2.64 (t, J = 11.7 Hz, 2 H), 2.49 (s, 5 H) 2.30 (t, J = 11.6 Hz, 4 H), 2.15 (s, 3 H), 1.84 (d, J = 11.8 Hz, 2 H), 1.51 (tt, J = 13.6, 6.9 Hz, 2 H), 1.20 (t, J = 7.3 Hz, 3 H).

According to the following reaction scheme, the compound of Example 14 was obtained.

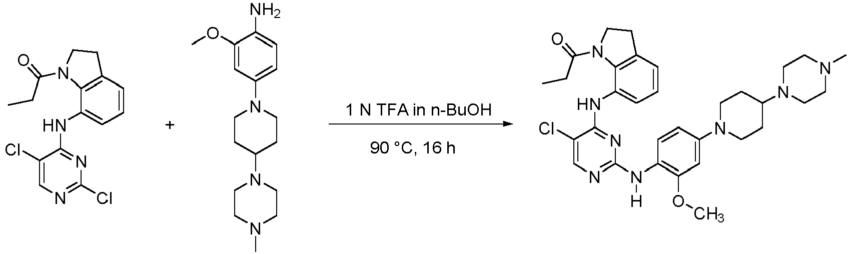

### <Example 14> Preparation of 1-(7-((5-chloro-2-((2-methoxy-4-(4-(4-methylpiperazin-1-yl)piperidin-1-yl)phenyl)amino)pyrimidin-4-yl)amino)indolin-1-yl)propan-1-one

The same method as in Example 1 was performed to obtain 1-(7-((5-chloro-2-((2-methoxy-4-(4-(4-methylpiperazin-1-yl)piperidin-1-yl)phenyl)amino)pyrimidin-4-yl)amino)indolin-1 -yl)propan-1 -one.

1H NMR (500 MHz, DMSO-d6) δ 9.88 (s, 1 H), 8.31 (s, 1 H), 8.06 (d, J = 8.1 Hz, 1 H), 7.93 (d, J = 8.6 Hz, 1 H), 7.72 (s, 1 H), 7.43 (d, J = 7.3 Hz, 1 H), 7.38 (d, J = 7.5 Hz, 1 H), 6.89 (d, J = 2.7 Hz, 1 H), 6.70 (dd, J = 8.7, 2.8 Hz, 1 H), 4.45 (t, J = 7.9 Hz, 2 H), 4.10 (s, 3 H), 3.95 (d, J = 12.3 Hz, 2 H), 3.41 (t, J = 7.8 Hz, 2 H), 3.05 - 2.97 (m, 2 H), 2.93 (q, J = 7.3 Hz, 2H), 2.85 (d, J = 4.7 Hz, 4 H), 2.68 (t, J = 4.8 Hz, 4 H), 2.55 (s, 3 H) 2.17 (d, J = 11.8 Hz, 2 H), 1.93 - 1.80 (m, 2 H), 1.74 (q, J = 6.9 Hz, 1 H), 1.65 (q, J = 7.4 Hz, 1 H), 1.47 (t, J = 7.3 Hz, 3 H).

According to the following reaction scheme, the compound of Example 15 was obtained.

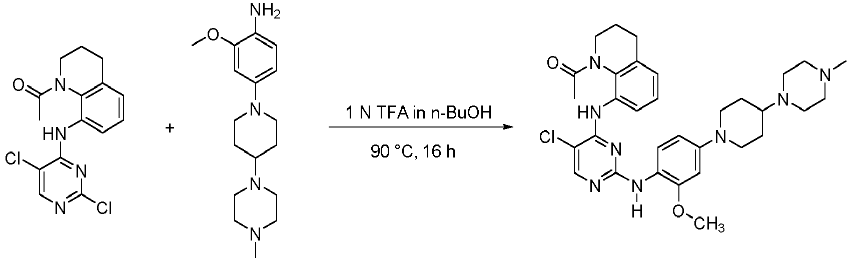

### <Example 15> Preparation of 1-(8-((5-chloro-2-((2-methoxy-4-(4-(4-methylpiperazin-1-yl)piperidin-1-yl)phenyl)amino)pyrimidin-4-yl)amino)-3,4-dihydroquinolin-1(2H)-yl)ethan-1-one

The same method as in Example 1 was performed to obtain 1-(8-((5-chloro-2-((2-methoxy-4-(4-(4-methylpiperazin-1-yl)piperidin-1-yl)phenyl)amino)pyrimidin-4-yl)amino)-3,4-dihydroquinolin-1(2H)-yl)ethan-1 -one.

¹H NMR (500 MHz, DMSO-d6) δ 8.02 (s, 1 H), 7.61 (d, J = 8.2 Hz, 1 H), 7.54 (d, J = 8.8 Hz, 1 H), 7.43 (s, 1 H), 7.21 (t, J = 7.8 Hz, 1 H), 7.08 (d, J = 7.6 Hz, 1 H), 6.57 (d, J = 2.6 Hz, 1 H), 6.34 (d, J = 8.4 Hz, 1 H), 3.79 (s, 3H ), 3.64 (d, J = 12.1 Hz, 2 H), 2.81 - 2.62 (m, 4 H), 2.57 - 2.52 (m, 4 H), 2.51 - 2.42 (m, 4 H), 2.39 - 2.26 (m, 5 H), 2.18 (s, 5 H), 1.95 (s, 2 H), 1.86 (d, J = 12.9 Hz, 2 H), 1.63 - 1.46 (m, 2H).

According to the following reaction scheme, the compound of Example 16 was obtained.

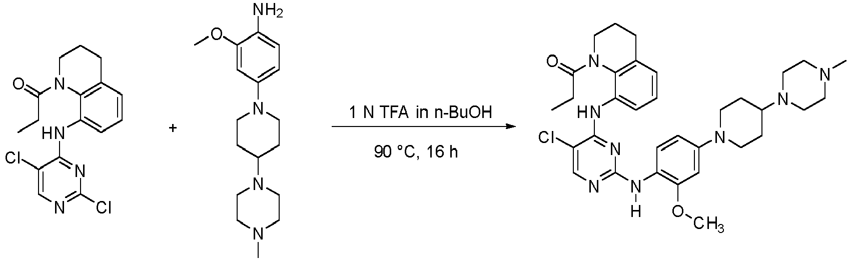

### <Example 16> Preparation of 1-(8-((5-chloro-2-((2-methoxy-4-(4-(4-methylpiperazin-1-yl)piperidin-1-yl)phenyl)amino)pyrimidin-4-yl)amino)-3,4-dihydroquinolin-1(2H)-yl)propan-1-one

The same method as in Example 1 was performed to obtain 1-(8-((5-chloro-2-((2-methoxy-4-(4-(4-methylpiperazin-1-yl)piperidin-1-yl)phenyl)amino)pyrimidin-4-yl)amino)-3,4-dihydroquinolin-1(2H)-yl)propan-1 -one.

¹H NMR (500 MHz, DMSO-d6) δ 7.99 (d, J = 7.7 Hz, 1 H), 7.92 (s, 1 H), 7.64 - 7.57 (m, 1 H), 7.55 - 7.48 (m, 1 H), 7.40 (d, J = 9.1 Hz, 1 H), 7.24 - 7.13 (m, 1 H), 7.07 (d, J = 8.2 Hz, 1 H), 6.55 (d, J = 8.6 Hz, 1 H), 6.32 (d, J = 8.9 Hz, 1 H), 3.78 (s, 3 H), 3.62 (s, 2 H), 2.68 (t, J = 11.1 Hz, 4 H), 2.37 - 2.26 (m, 6 H), 2.17 (s, 3 H), 1.92 (s, 2 H), 1.85 (d, J = 11.7 Hz, 2 H), 1.54 (t, J = 11.6 Hz, 2 H), 1.13 - 0.98 (m, 3 H).

According to the following reaction scheme, the compound of Example 17 was obtained.

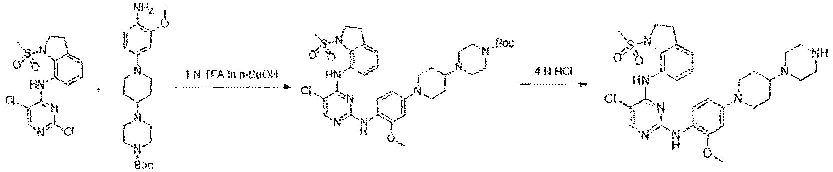

### <Example 17> Preparation of 5-chloro-N2-(2-methoxy-4-(4-(piperazin-1-yl)piperidin-1-yl)phenyl)-N4-(1-(methylsulfonyl)indolin-7-yl)pyrimidine-2,4-diamine

The same method as in Example 1 was performed to obtain the intermediate compound tert-butyl 4-(1-(4-((5-chloro-4-((1-(methylsulfonyl)indolin-7-yl)amino)pyrimidin-2-yl)amino)-3-methoxyphenyl)piperidin-4-yl)piperazine-1-carboxylate. Next, the deprotection reaction was performed using 4N HCl to obtain the target compound 5-chloro-N2-(2-methoxy-4-(4-(piperazin-1-yl)piperidin-1-yl)phenyl)-N4-(1-(methylsulfonyl)indolin-7-yl)pyrimidine-2,4-diamine.

¹H NMR (500 MHz, Methanol-d4) δ 8.10 (s, 1 H), 7.71 (d, J = 7.9 Hz, 1 H), 7.48 (d, J = 8.6 Hz, 1 H), 7.36 (d, J = 7.6 Hz, 1 H), 7.30 (t, J = 7.8 Hz, 1 H), 6.88 (s, 1H), 6.67 (d, J = 8.7 Hz, 1 H), 4.13 (t, J = 7.6 Hz, 2 H), 3.94 - 3.84 (m, 5H), 3.56 (d, J = 5.1 Hz, 4 H), 3.50 (s, 4 H), 3.37 (m, 1 H), 3.20 (t, J = 7.5 Hz, 2 H), 3.09 (t, J = 12.5 Hz, 2 H), 2.98 (s, 3 H), 2.28 (d, J = 12.3 Hz, 2 H), 2.05 - 1.95 (m, 2 H).

Table 1 below summarizes the structural formulas of the compounds prepared in Examples 1-17.

**[Table 1]**

| Example | Chemical Structure | Example | Chemical Structure |
|---|---|---|---|
| 1 | 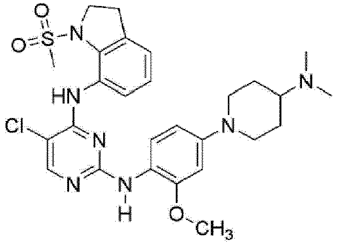 | 2 | 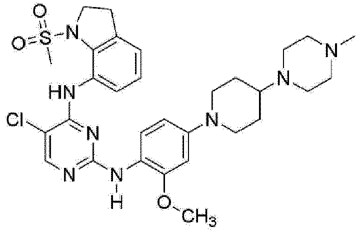 |
| 3 | 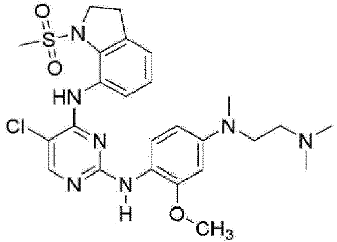 | 4 | 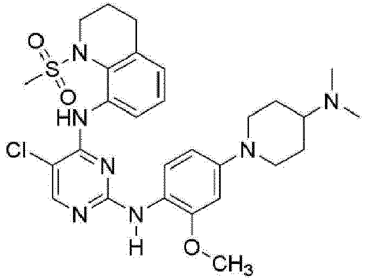 |
| 5 | 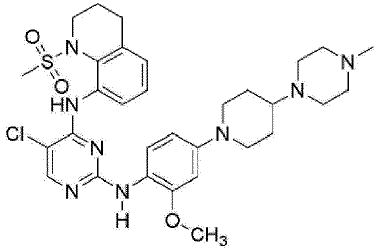 | 6 | 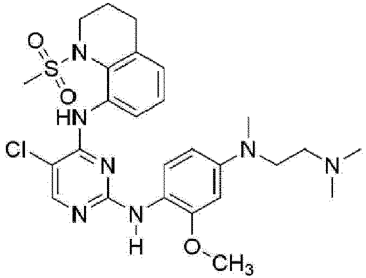 |
| 7 | 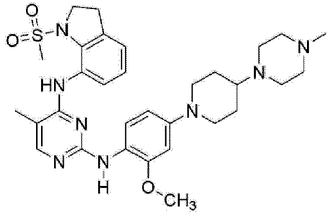 | 8 | 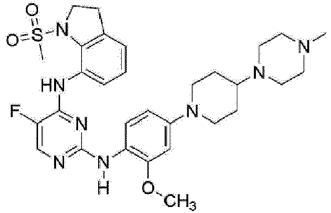 |
| 9 | 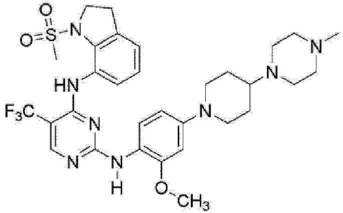 | 10 | 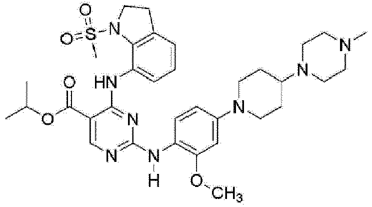 |
| 11 | 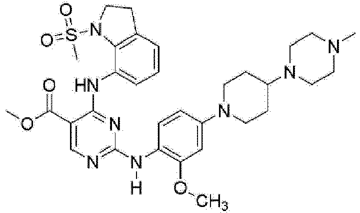 | 12 | 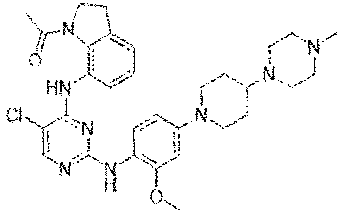 |
| 13 | 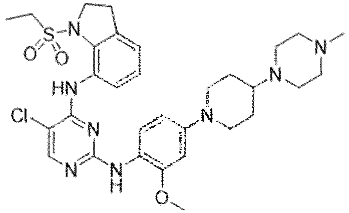 | 14 | 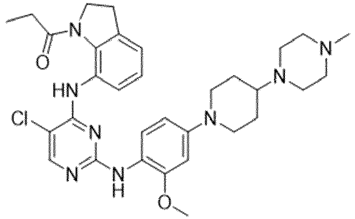 |
| 15 | 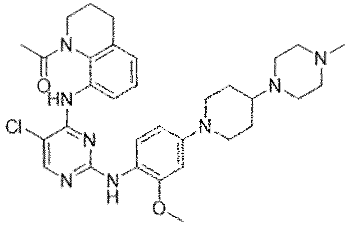 | 16 | 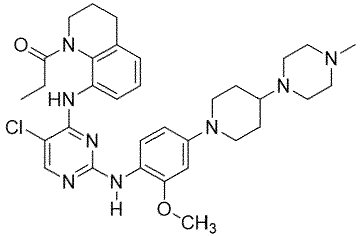 |
| 17 | 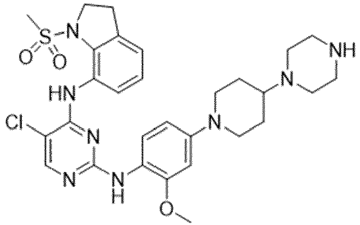 | | |

### <Experimental Example 1> Measurement of inhibitory ability of the compounds represented by Formula 1 according to the present invention against wild type EGFR and EGFR mutants

In order to confirm the inhibitory ability of the compounds represented by Formula 1 according to the present invention against various EGFR mutations, the following experiment was performed. The results are shown in Table 2 below.

An experiment to measure the activity of the compounds of the present invention against EGFR mutant enzymes was performed as follows using the HTRF system sold by Cisbio. For the EGFR del19/T790M mutant enzyme, a recombinant protein provided by Carna Biosciences was purchased and used, and for the EGFR del19/T790M/C797S mutant enzyme, a protein provided by SignalChem was purchased and used as an enzyme source.

The composition of the assay buffer used for measuring the activity was 50 mM Tris-HCl pH 7.5, 100 mM NaCl, 7.5 mM MgCl₂, 3 mM KCl, 0.01% Tween 20, 0.1% BSA, and 1 mM DTT. Here, an enzyme reaction was performed using ATP at a concentration of 50 mM and a peptide substrate labeled with biotin at a concentration of 0.5 mM. Analysis of the inhibitory effect of the compounds against the EGFR activity was conducted according to the following analysis reaction recipe.
Component 1: 4 µL of an EGFR mutant enzyme
Component 2: 2 µL of a compound solution
Component 3: 4 µL of ATP and a peptide labeled with biotin

The enzyme reaction was started by first mixing Component 1 and Component 2 and then adding Component 3. After reaction at 37°C for 2 hours, 10 mL of measurement solution consisting of streptavidin-XL665 and europium-labeled anti-phosphotyrosine antibody provided by Cisbio was added to the enzyme reaction solution and reacted at room temperature for 1 hour. Finally, the ratio of fluorescence values at 615 nm and 665 nm was obtained using the Envision equipment from Perkin-Elmer to quantitatively measure the enzyme activity and confirmed the inhibitory ability of the compounds. The measurement values measured at seven compound concentrations were analyzed using the Prism program (version 5.01, Graphpad Software, Inc.), and the IC₅₀ value, which is an indicator of the inhibitory ability of the compound, was calculated.

**[Table 2]**

| Example | EGFR del19/T790M IC₅₀(µM) | EGFR del19/T790M/C797S IC₅₀(µM) |
|---|---|---|
| 1 | <0.001 | <0.001 |
| 2 | <0.001 | <0.001 |
| 3 | <0.001 | <0.001 |
| 4 | <0.001 | <0.001 |
| 5 | <0.001 | <0.001 |
| 6 | 0.001 | <0.001 |
| 7 | <0.001 | <0.001 |
| 8 | <0.001 | <0.001 |
| 9 | <0.001 | <0.001 |
| 10 | 0.06 | 0.05 |
| 11 | 0.002 | 0.005 |
| 12 | <0.001 | <0.001 |
| 13 | <0.001 | <0.001 |
| 14 | 0.091 | 0.050 |
| 15 | 0.073 | 0.056 |
| 16 | 0.170 | 0.150 |
| 17 | <0.001 | <0.001 |

As shown in Table 2 above, it can be seen that the example compounds of the present invention exhibit a high inhibitory ability against the EGFR double and triple mutations, EGFR del19/T790M and EGFR del19/T790M/C797S.

Therefore, the compounds represented by Formula 1 of the present invention have an excellent inhibitory effect against the EGFR double and triple mutations, EGFR del19/T790M and EGFR del19/T790M/C797S, and thus can be advantageously used in the treatment of cancer, a disease related to EGFR mutations, and in particular, it can be advantageously used in the treatment of cancer expressing EGFR del19/T790M/C797S.

### <Experimental Example 2> Measurement of inhibitory ability of the compounds represented by Formula 1 according to the present invention against EGFR mutation in Ba/F3 cell line

In order to confirm the inhibitory ability of the compounds represented by Formula 1 according to the present invention against EGFR mutation in Ba/F3 cell line, the following experiment was performed. The results are shown in Table 3 below.

An experiment to measure the activity of the compounds of the present invention against the mutant Ba/F3 EGFR cell line was performed as follows using the CellTiter-Glo system sold by Promega. The CellTiter-Glo assay is a method to confirm the cell viability by measuring ATP present in cells in cell culture. For the Ba/F3 EGFR del19, Ba/F3 EGFR del19/T790M, Ba/F3 del19/T790M/C797S mutant cell line, a cell line provided by Crown Bioscience was purchased and used. The Ba/F3 EGFR del19, Ba/F3 EGFR del19/T790M, Ba/F3 del19/T790M/C797S mutant cell line was cultured in RPMI containing 10% FBS and 1% penicillin-streptomycin with 1 µg of puromycin in an incubator at 37°C and 5% CO₂.

Analysis of the inhibitory ability effect of the compounds against EGFR was conducted according to the following analysis reaction recipe.

2500 cells/90 µlL were passaged and cultured in a 96-well cell culture plate, and after 24 hours, treated with the compound represented by Formula 1 at a concentration of 0, 0.01, 0.03, 0.1, 0.3, 1, 3, and 10 (µM). After reaction for 72 hours, the plate treated with the compound was left at room temperature for 30 minutes, then further treated with 100 µl of reagent, and shaken at room temperature for 10 minutes. Finally, the ratio of fluorescence values at 570 nm was obtained using the equipment to quantitatively measure and confirmed the inhibitory ability of the compounds. The measurement values measured at eight compound concentrations were analyzed using the Prism program (version 5.01, Graphpad Software, Inc.), and the IC₅₀ value, which is an indicator of the inhibitory ability of the compound, was calculated.

**[Table 3]**

| Example | Ba/F3 EGFR del 19 IC₅₀(µM) | Ba/F3 EGFR del19/T790M IC₅₀(µM) | Ba/F3 EGFR del19/T790M/C797S IC₅₀(µM) |
|---|---|---|---|
| 1 | <0.030 | <0.030 | <0.100 |
| 2 | <0.030 | <0.100 | <0.100 |
| 3 | <0.100 | <0.100 | >0.100 |
| 4 | <0.030 | <0.030 | <0.030 |
| 5 | <0.030 | <0.030 | <0.030 |
| 6 | <0.100 | <0.100 | >0.100 |
| 7 | <0.030 | <0.100 | >0.100 |
| 8 | <0.100 | >0.100 | >0.100 |
| 9 | <0.030 | <0.030 | >0.100 |
| 10 | >0.100 | >0.100 | >0.100 |
| 11 | <0.100 | >0.100 | >0.100 |
| 12 | <0.100 | >0.100 | >0.100 |
| 13 | <0.100 | >0.100 | >0.100 |
| 14 | <0.100 | >0.100 | >0.100 |
| 15 | >0.100 | >0.100 | >0.100 |
| 16 | >0.100 | >0.100 | >0.100 |
| 17 | <0.100 | <0.100 | >0.100 |

As shown in Table 3, it was confirmed that the example compounds according to the present invention exhibit a high inhibitory ability against various EGFR mutations, including the triple mutation EGFR del19/T790M/C797S.

### <Experimental Example 3> Measurement 2 of inhibitory ability of the compounds represented by Formula 1 according to the present invention against EGFR mutation in Ba/F3 cell line

In order to confirm the inhibitory ability of the compounds represented by Formula 1 according to the present invention against EGFR mutation in Ba/F3 cell line, the following experiment was further performed. The results are shown in Table 4 below.

An experiment to measure the activity of the compounds of the present invention against the mutant Ba/F3 EGFR cell line was performed as follows using the CellTiter-Glo system sold by Promega. The CellTiter-Glo assay is a method to confirm the cell viability by measuring ATP present in cells in cell culture. The Ba/F3 EGFR L858R, Ba/F3 EGFR L858R/T790M, Ba/F3 EGFR L858R/T790M/C797S mutant cell line was used. The Ba/F3 EGFR L858R, Ba/F3 EGFR L858R/T790M, and Ba/F3 EGFR L858R/T790M/C797S mutant cell lines was cultured in RPMI containing 10% FBS and 1% penicillin-streptomycin with 1 µg of puromycin in an incubator at 37°C and 5% CO₂.

Analysis of the inhibitory ability effect of the compounds against EGFR was conducted according to the following analysis reaction recipe.

2500 cells/90 µl were passaged and cultured in a 96-well cell culture plate, and after 24 hours, treated with the compound represented by Formula 1 at a concentration of 0, 0.01, 0.03, 0.1, 0.3, 1, 3, and 10 (µM). After reaction for 72 hours, the plate treated with the compound was left at room temperature for 30 minutes, then further treated with 100 µl of reagent, and shaken at room temperature for 10 minutes. Finally, the ratio of fluorescence values at 570 nm was obtained using the equipment to quantitatively measure and confirmed the inhibitory ability of the compounds. The measurement values measured at eight compound concentrations were analyzed using the Prism program (version 5.01, Graphpad Software, Inc.), and the IC₅₀ value, which is an indicator of the inhibitory ability of the compound, was calculated.

**[Table 4]**

| Example | Ba/F3 EGFR L858R IC₅₀(µM) | Ba/F3 EGFR L858R/T790M IC₅₀(µM) | Ba/F3 EGFR L858R/T790M/C797S IC₅₀(µM) |
|---|---|---|---|
| 1 | <0.030 | <0.030 | <0.100 |
| 2 | <0.030 | <0.030 | <0.100 |
| 3 | >0.100 | >0.100 | na |
| 4 | <0.100 | <0.100 | na |
| 5 | <0.030 | <0.030 | na |
| 6 | >0.100 | <0.100 | na |
| 7 | <0.030 | <0.100 | >0.100 |
| 8 | <0.030 | <0.100 | >0.100 |
| 9 | <0.100 | <0.030 | >0.100 |
| 10 | <0.100 | >0.100 | >0.100 |
| 11 | <0.100 | <0.100 | >0.100 |
| 12 | >0.100 | >0.100 | >0.100 |
| 13 | >0.100 | >0.100 | >0.100 |
| 14 | >0.100 | >0.100 | >0.100 |
| 15 | >0.100 | >0.100 | >0.100 |
| 16 | >0.100 | >0.100 | >0.100 |
| 17 | <0.100 | >0.100 | >0.100 |

As shown in Table 4, it was confirmed that the example compounds according to the present invention exhibit a high inhibitory ability against various EGFR mutations, including the triple mutation Ba/F3 EGFR L858R/T790M/C797S.

Therefore, the compounds represented by Formula 1 according to the present invention exhibit a high inhibitory ability against the EGFR mutations, and thus can be advantageously used in the treatment of cancer expressing EGFR mutations such as EGFR del19, EGFR del19/T790M, EGFR del19/T790M/C797S, EGFR L858R, EGFR L858R/T790MS, and EGFR L858R/T790M/C797S. In particular, these compounds exhibit a significantly excellent inhibitory ability against the triple mutation EGFR del19/T790M/C797S or EGFR L858R/T790M/C797S. Accordingly, the compounds represented by Formula 1 according to the present invention can also be advantageously used in the treatment of cancer expressing EGFR del19/T790M/C797S or EGFR L858R/T790M/C797S.

In addition, when administered in combination with the conventional drug, the compounds represented by Formula 1 according to the present invention exhibit a synergistic effect, and thus can also be advantageously used in combination with the conventional drug.

### <Experimental Example 4> Measurement of inhibitory ability of the compounds represented by Formula 1 according to the present invention against EGFR Ex20 insertion mutant enzyme

In order to confirm the inhibitory ability of the compounds represented by Formula 1 according to the present invention against EGFR Ex20 insertion mutant enzyme, the following experiment was performed. The results are shown in Table 5 below.

An experiment to measure the activity of the compounds of the present invention against EGFR mutant enzymes was performed as follows using the HTRF system sold by Cisbio. For the EGFR mutant enzyme, EGFR A763_Y764insFHEA enzyme, a recombinant protein provided by SignalChem was purchased and used.

The composition of the assay buffer used for measuring the activity was 50 mM Tris-HCl pH 7.5, 100 mM NaCl, 7.5 mM MgCl₂, 3 mM KCl, 0.01% Tween 20, 0.1% BSA, and 1 mM DTT. Here, an enzyme reaction was performed using ATP at a concentration of 50 mM and a peptide substrate labeled with biotin at a concentration of 0.5 mM. Analysis of the inhibitory effect of the compounds against the EGFR activity was conducted according to the following analysis reaction recipe.
Component 1: 4 µL of an EGFR mutant enzyme
Component 2: 2 µL of a compound solution
Component 3: 4 µL of ATP and a peptide labeled with biotin

The enzyme reaction was started by first mixing Component 1 and Component 2 and then adding Component 3. After reaction at 37°C for 2 hours, 10 mL of measurement solution consisting of streptavidin-XL665 and europium-labeled anti-phosphotyrosine antibody provided by Cisbio was added to the enzyme reaction solution and reacted at room temperature for 1 hour. Finally, the ratio of fluorescence values at 615 nm and 665 nm was obtained using the Envision equipment from Perkin-Elmer to quantitatively measure the enzyme activity and confirmed the inhibitory ability of the compounds. The measurement values measured at seven compound concentrations were analyzed using the Prism program (version 5.01, Graphpad Software, Inc.), and the IC₅₀ value, which is an indicator of the inhibitory ability of the compound, was calculated.

**[Table 5]**

| Example | EGFR A763_Y764insFHEA IC₅₀ (µM) |
|---|---|
| 2 | 0.0017 |
| 5 | 0.011 |
| 12 | 0.178 |
| 13 | 0.091 |
| 14 | > 0.1 |
| 15 | > 0.1 |
| 16 | > 0.1 |
| 17 | 0.020 |

As shown in Table 5, it was confirmed that the example compounds according to the present invention exhibit a high inhibitory ability against the EGFR Ex20 insertion mutation, EGFR A763_Y764insFHEA mutation.

### <Experimental Example 5> Measurement of inhibitory ability of the compounds represented by Formula 1 according to the present invention against Ba/F3 EGFR Ex20 insertion mutant cell growth

In order to confirm the inhibitory ability of the compounds represented by Formula 1 according to the present invention against Ba/F3 EGFR Ex20 insertion mutant cell growth, the following experiment was performed. The results are shown in Table 6 below.

An experiment to measure the activity of the compounds of the present invention against the Ba/F3 EGFR V769_D770insASV mutant cell line was performed as follows using the CellTiter-Glo system sold by Promega. The CellTiter-Glo assay is a method to confirm the cell viability by measuring ATP present in cells in cell culture. For the Ba/F3 EGFR V769_D770insASV mutant cell line, a cell line provided by Crown Bioscience was purchased and used. The Ba/F3 EGFR V769_D770insASV mutant cell line was cultured in RPMI containing 10% FBS and 1% penicillin-streptomycin with 1 µg/ml of puromycin in an incubator at 37°C and 5% CO₂.

Analysis of the inhibitory ability effect of the compounds against the EGFR mutant cell growth was conducted according to the following analysis reaction recipe.

1000 cells/100 µL were passaged and cultured in a 96-well cell culture plate, and after 24 hours, treated with the compound represented by Formula 1 at a concentration of 0, 0.01, 0.03, 0.1, 0.3, 1, 3, and 10 (µM). After reaction for 72 hours, the plate treated with the compound was left at room temperature for 30 minutes, then further treated with 100 µL of reagent, and shaken at room temperature for 10 minutes. Finally, the ratio of fluorescence values at 570 nm was obtained using the equipment to quantitatively measure and confirmed the cell growth inhibitory ability of the compounds. The measurement values measured at eight compound concentrations were analyzed using the Prism program (version 5.01, Graphpad Software, Inc.), and the IC₅₀ value, which is an indicator of the cell growth inhibitory ability of the compound, was calculated.

**[Table 6]**

| Example | Ba/F3 EGFR V769 _D770insASV IC₅₀ (µM) |
|---|---|
| 2 | 0.21 |
| 5 | 0.36 |
| 12 | >0.100 |
| 13 | >0.100 |
| 14 | >0.100 |
| 15 | >0.100 |
| 16 | >0.100 |
| 17 | >0.100 |

As shown in Table 6, it was confirmed that the example compounds according to the present invention exhibit a high inhibitory ability against the Ba/F3 EGFR V769_D770insASV mutant cell growth.

### <Experimental Example 6> Measurement of inhibitory ability of the compounds represented by Formula 1 according to the present invention against HER2 mutation

In order to confirm the inhibitory ability of the compounds represented by Formula 1 according to the present invention against HER2 mutation, the following experiment was performed. The results are shown in Table 7 below.

An experiment to measure the activity of the compounds of the present invention against HER mutant enzymes was performed as follows using the HTRF system sold by Cisbio. For the HER2 mutant enzyme, HER2 A775_G776insYVMA mutant enzyme, a recombinant protein provided by Carna Biosciences was purchased and used.

The composition of the assay buffer used for measuring the activity was 50 mM Tris-HCl pH 7.5, 100 mM NaCl, 7.5 mM MgCl₂, 3 mM KCl, 0.01% Tween 20, 0.1% BSA, and 1 mM DTT. Here, an enzyme reaction was performed using with ATP at a concentration of 50 mM and a peptide substrate labeled biotin at a concentration of 0.5 mM. Analysis of the inhibitory effect of the compounds against the HER2 A775_G776insYVMA mutant activity was conducted according to the following analysis reaction recipe.
Component 1: 4 µL of a HER2 A775_G776insYVMA mutant enzyme
Component 2: 2 µL of a compound solution
Component 3: 4 µL of ATP and a peptide labeled with biotin

The enzyme reaction was started by first mixing Component 1 and Component 2 and then adding Component 3. After reaction at 37°C for 2 hours, 10 mL of measurement solution consisting of streptavidin-XL665 and europium-labeled anti-phosphotyrosine antibody provided by Cisbio was added to the enzyme reaction solution and reacted at room temperature for 1 hour. Finally, the ratio of fluorescence values at 615 nm and 665 nm was obtained using the Envision equipment from Perkin-Elmer to quantitatively measure the enzyme activity and confirmed the inhibitory ability of the compounds. The measurement values measured at seven compound concentrations were analyzed using the Prism program (version 5.01, Graphpad Software, Inc.), and the IC₅₀ value, which is an indicator of the inhibitory ability of the compound, was calculated.

**[Table 7]**

| Example | HER2 A775_G776insYVMA IC₅₀ (µM) |
|---|---|
| 2 | 0.001 |
| 5 | 0.003 |
| 12 | 0.025 |
| 13 | 0.026 |
| 14 | > 0.1 |
| 15 | 0.589 |
| 16 | > 0.1 |
| 17 | 0.004 |

As shown in Table 7, it was confirmed that the example compounds according to the present invention exhibit a high inhibitory ability against the HER2 A775_G776insYVMA mutation. Therefore, the compounds represented by Formula 1 according to the present invention exhibit a high inhibitory ability against the EGFR mutation, and thus can be advantageously used in the treatment of cancer expressing EGFR mutations such as EGFR del19/T790M, EGFR del19/T790M/C797S and EGFR A763_Y764insFHEA, Ba/F3 EGFR del19, Ba/F3 EGFR del19/T790M, Ba/F3 EGFR del19/T790M/C797S, Ba/F3 EGFR L858R, Ba/F3 EGFR L858R/T790M, Ba/F3 EGFR L858R/T790M/C797S, Ba/F3 EGFR A763_Y764insFHEA, Ba/F3 EGFR V769_D770insASV and Ba/F3 EGFR D770_N771insSVD.

In addition, the compounds represented by Formula 1 according to the present invention exhibit a high inhibitory ability against the HER2 mutations, and thus can also be advantageously used in the treatment of cancer expressing HER2 mutations such as HER2 A775_G776insYVMA, Ba/F3 HER2 A775_G776insYVMA, and Ba/F3 HER2 G776_delinsVC.

From the above results, it can be seen that the pyrimidine-2,4-diamine derivative compound according to the present invention can effectively inhibit EGFR and HER2 mutations, and thus can be advantageously used as a pharmaceutical composition for preventing or treating cancer.

## Claims

1. A compound represented by Formula 1 below, a stereoisomer thereof, a solvate thereof, a hydrate thereof, or a pharmaceutically acceptable salt thereof: 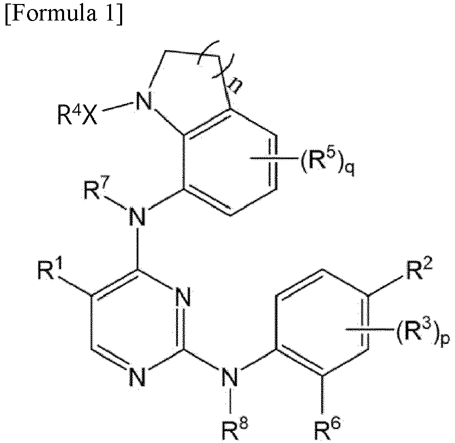 in Formula 1 above,
X is sulfonyl or carbonyl;
R¹ is hydrogen, halogen, nitrile, a straight chain or branched chain C₁₋₁₀ alkyl unsubstituted or substituted with one or more halogens, carboxyl, or a straight chain or branched chain C₁₋₁₀ alkoxycarbonyl;
R² is -ORₐ or -NR_{b1}R_{b2},
Rₐ is -(CH₂)ₘ-NR_{b1}R_{b2}, wherein m is an integer from 1 to 3,
R_{b1} and R_{b2} are each independently hydrogen, an unsubstituted or substituted straight chain or branched chain C₁₋₁₀ alkyl, or R_{b1} and R_{b2} are taken together with the nitrogen to which they are attached to form an unsubstituted or substituted 3 to 7-membered heterocycloalkyl, wherein the substituent of the substituted straight chain or branched chain C₁₋₁₀ alkyl may be - NR_{c1}R_{c2}, and the substituent of the substituted 3 to 7-membered heterocycloalkyl may be - NR_{c1}R_{c2} or a straight chain or branched chain C₁₋₁₀ alkyl, and
R_{c1} and R_{c2} are each independently hydrogen, a straight chain or branched chain C₁₋₁₀ alkyl, or R_{c1} and R_{c2} are taken together with the nitrogen to which they are attached to form an unsubstituted or substituted 3 to 7-membered heterocycloalkyl, wherein the substituent of the substituted 3 to 7-membered heterocycloalkyl may be a straight chain or branched chain C₁₋₁₀ alkyl; and
R³, R⁵ and R⁶ are each independently hydrogen, halogen, a straight chain or branched chain C₁₋₁₀alkoxy, or a straight chain or branched chain C₁₋₁₀ alkyl unsubstituted or substituted with one or more halogens;
R⁴ is -NH₂, a straight chain or branched chain C₁₋₁₀ alkyl, or C₃₋₇ cycloalkyl;
R⁷ and R⁸ are each independently hydrogen or a straight chain or branched chain C₁₋₁₀ alkyl;
n is an integer from 0 to 3; and
p and q are each independently an integer from 1 to 3.

2. The compound, stereoisomer thereof, solvate thereof, hydrate thereof, or pharmaceutically acceptable salt thereof according to claim 1, wherein
R¹ is hydrogen, halogen, a straight chain or branched chain C₁₋₃ alkyl unsubstituted or substituted with one or more halogens, or a straight chain or branched chain C₁₋₃ alkoxycarbonyl;
R² is -NR_{b1}R_{b2},
R_{b1} and R_{b2} are each independently hydrogen, an unsubstituted or substituted straight chain or branched chain C₁₋₃ alkyl, or R_{b1} and R_{b2} are taken together with the nitrogen to which they are attached to form an unsubstituted or substituted 5 to 6-membered heterocycloalkyl, wherein the substituent of the substituted straight chain or branched chain C₁₋₃ alkyl may be - NR_{c1}R_{c2}, and the substituent of the substituted 5 to 6-membered heterocycloalkyl may be - NR_{c1}R_{c2} or a straight chain or branched chain C₁₋₃ alkyl, and
R_{c1} and R_{c2} are each independently hydrogen, a straight chain or branched chain C₁₋₃ alkyl, or R_{c1} and R_{c2} are taken together with the nitrogen to which they are attached to form an unsubstituted or substituted 5 to 6-membered heterocycloalkyl, wherein the substituent of the substituted 5 to 6-membered heterocycloalkyl may be a straight chain or branched chain C₁₋₃ alkyl; and
R³ and R⁵ are each independently hydrogen, or a straight chain or branched chain C₁₋₃ alkyl unsubstituted or substituted with one or more halogens;
R⁴ is -NH₂, a straight chain or branched chain C₁₋₃ alkyl, or C₄₋₆ cycloalkyl;
R⁶ is a straight chain or branched chain C₁₋₃ alkoxy; and
n is an integer from 1 to 3.

3. The compound, stereoisomer thereof, solvate thereof, hydrate thereof, or pharmaceutically acceptable salt thereof according to claim 1, wherein
R¹ is hydrogen, halogen, C₁₋₂ alkyl unsubstituted or substituted with one or more halogens, or C₁₋₃ alkoxycarbonyl;
R² is -NR_{b1}R_{b2},
R_{b1} and R_{b2} are each independently hydrogen, unsubstituted or substituted C₁₋₂ alkyl, or R_{b1} and R_{b2} are taken together with the nitrogen to which they are attached to form an unsubstituted or substituted 6-membered heterocycloalkyl, wherein the substituent of the substituted C₁₋₂ alkyl may be -NR_{c1}R_{c2}, and the substituent of the substituted 6-membered heterocycloalkyl may be -NR_{c1}Rₑ₂ or C₁₋₂ alkyl, and
R_{c1} and R_{c2} are each independently hydrogen, C₁₋₂ alkyl, or R_{c1} and R_{c2} are taken together with the nitrogen to which they are attached to form an unsubstituted or substituted 6-membered heterocycloalkyl, wherein the substituent of the substituted 6-membered heterocycloalkyl may be C₁₋₂ alkyl; and
R³ and R⁵ are each independently hydrogen or C₁₋₂ alkyl;
R⁴ is C₁₋₂ alkyl;
R⁶ is methoxy;
R⁷ and R⁸ are each independently hydrogen or C₁₋₂ alkyl; and
n is 1 or 2.

4. The compound, stereoisomer thereof, solvate thereof, hydrate thereof, or pharmaceutically acceptable salt thereof according to claim 1, wherein
R¹ is methyl, F, Cl, CF₃, 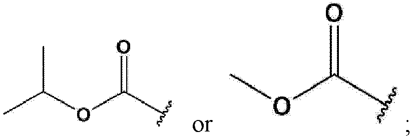
R² is 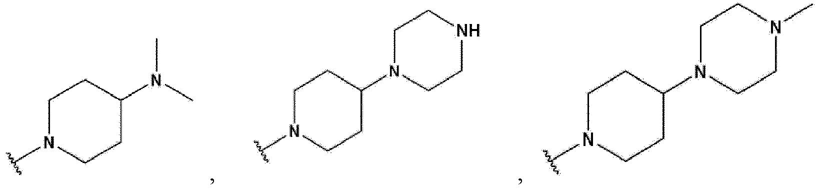 or 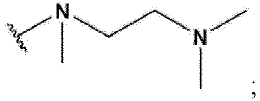
R³ is hydrogen;
R⁴ is methyl or ethyl;
R⁵ is hydrogen;
R⁶ is methoxy;
R⁷ is hydrogen;
R⁸ is hydrogen; and
n is 1 or 2.

5. The compound, stereoisomer thereof, solvate thereof, hydrate thereof, or pharmaceutically acceptable salt thereof according to claim 1, wherein the compound represented by Formula 1 is any one compound selected from the group consisting of the following compounds:
<1> 5-chloro-N2-(4-(4-(dimethylamino)piperidin-1-yl)-2-methoxyphenyl)-N4-(1-(methylsulfonyl)indolin-7-yl)pyrimidine-2,4-diamine;
<2> 5-chloro-N2-(2-methoxy-4-(4-(4-methylpiperazin-1-yl)piperidin-1-yl)phenyl)-N4-(1-(methylsulfonyl)indolin-7-yl)pyrimidine-2,4-diamine;
<3> 5-chloro-N2-(4-((2-(dimethylamino)ethyl)(methyl)amino)-2-methoxyphenyl)-N4-(1-(methylsulfonyl)indolin-7-yl)pyrimidine-2,4-diamine;
<4> 5-chloro-N2-(4-(4-(dimethylamino)piperidin-1-yl)-2-methoxyphenyl)-N4-(1-(methylsulfonyl)-1,2,3,4-tetrahydroquinolin-8-yl)pyrimidine-2,4-diamine;
<5> 5-chloro-N2-(2-methoxy-4-(4-(4-methylpiperazin-1-yl)piperidin-1-yl)phenyl)-N4-(1-(methylsulfonyl)-1,2,3,4-tetrahydroquinolin-8-yl)pyrimidine-2,4-diamine;
<6> 5-chloro-N2-(4-((2-(dimethylamino)ethyl)(methyl)amino)-2-methoxyphenyl)-N4-(1-(methylsulfonyl)-1,2,3,4-tetrahydroquinolin-8-yl)pyrimidine-2,4-diamine;
<7> N2-(2-methoxy-4-(4-(4-methylpiperazin-1-yl)piperidin-1-yl)phenyl)-5-methyl-N4-(1-(methylsulfonyl)indolin-7-yl)pyrimidine-2,4-diamine;
<8> 5-fluoro-N2-(2-methoxy-4-(4-(4-methylpiperazin-1-yl)piperidin-1-yl)phenyl)-N4-(1-(methylsulfonyl)indolin-7-yl)pyrimidine-2,4-diamine;
<9> N2-(2-methoxy-4-(4-(4-methylpiperazin-1-yl)piperidin-1-yl)phenyl)-N4-(1-(methylsulfonyl)indolin-7-yl)-5-(trifluoromethyl)pyrimidine-2,4-diamine;
<10> isopropyl 2-((2-methoxy-4-(4-(4-methylpiperazin-1-yl)piperidin-1-yl)phenyl)amino)-4-((1-(methylsulfonyl)indolin-7-yl)amino)pyrimidine-5-carboxylate;
<11> methyl 2-((2-methoxy-4-(4-(4-methylpiperazin-1-yl)piperidin-1-yl)phenyl)amino)-4-((1-(methylsulfonyl)indolin-7-yl)amino)pyrimidine-5-carboxylate;
<12> 1-(7-((5-chloro-2-((2-methoxy-4-(4-(4-methylpiperazin-1-yl)piperidin-1-yl)phenyl)amino) pyrimidin-4-yl)amino)indolin-1-yl)ethan-1-one;
<13> 5-chloro-N4-(1-(ethylsulfonyl)indolin-7-yl)-N2-(2-methoxy-4-(4-(4-methylpiperazin-1-yl)piperidin-1-yl)phenyl)pyrimidine-2,4-diamine;
<14> 1-(7-((5-chloro-2-((2-methoxy-4-(4-(4-methylpiperazin-1-yl)piperidin-1-yl)phenyl)amino)pyrimidin-4-yl)amino)indolin-1-yl)propan-1-one;
<15> 1-(8-((5-chloro-2-((2-methoxy-4-(4-(4-methylpiperazin-1-yl)piperidin-1-yl)phenyl)amino)pyrimidin-4-yl)amino)-3,4-dihydroquinolin-1 (2H)-yl)ethan-1-one;
<16> 1-(8-((5-chloro-2-((2-methoxy-4-(4-(4-methylpiperazin-1-yl)piperidin-1-yl)phenyl)amino)pyrimidin-4-yl)amino)-3,4-dihydroquinolin-1(2H)-yl)propan-1-one; and
<17> 5-chloro-N2-(2-methoxy-4-(4-(piperazin-1-yl)piperidin-1-yl)phenyl)-N4-(1-(methylsulfonyl)indolin-7-yl)pyrimidine-2,4-diamine.

6. A method for preparing the compound represented by Formula 1 according to claim 1, comprising:
reacting a compound represented by Formula 2 with a compound represented by Formula 3 to prepare a compound represented by Formula 1, as shown in Reaction Scheme 1 below: 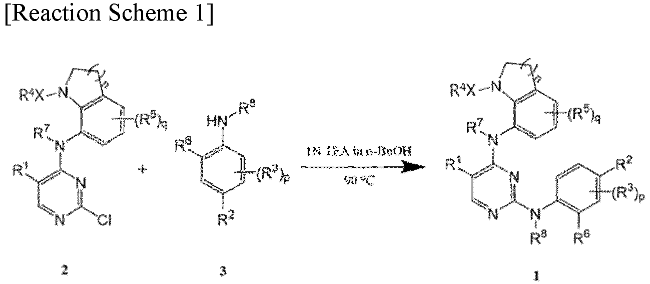
in Reaction Scheme 1 above, X, R¹, R², R³, R⁴, R⁵, R⁶, R⁷, R⁸, n, p and q are as defined in Formula 1 according to claim 1.

7. A pharmaceutical composition for preventing or treating cancer, containing the compound represented by Formula 1, stereoisomer thereof, solvate thereof, hydrate thereof, or pharmaceutically acceptable salt thereof according to claim 1 as an active ingredient.

8. The pharmaceutical composition, wherein the compound represented by Formula 1, stereoisomer thereof, solvate thereof, hydrate thereof, or pharmaceutically acceptable salt thereof according to claim 1 inhibits EGFR (epidermal growth factor receptor) or HER2 mutations.

9. The pharmaceutical composition according to claim 8, wherein
the EGFR mutation is at least one selected from the group consisting of EGFR del19, EGFR T790M, EGFR C797S, EGFR L858R, EGFR Ex20 insertion mutations, EGFR A763_Y764insFHEA, EGFR V769_D770insASV and EGFR D770_N771insSVD, etc.; and
the HER2 mutation is at least one selected from the group consisting of HER2 A775_G776insYVMA, HER2 G776_delinsVC, etc.

10. The pharmaceutical composition according to claim 7, wherein the cancer is at least one selected from the group consisting of pseudomyxoma, intrahepatic biliary tract cancer, hepatoblastoma, liver cancer, thyroid cancer, colon cancer, testicular cancer, myelodysplastic syndrome, glioblastoma, oral cancer, lip cancer, mycosis fungoides, acute myeloid leukemia, acute lymphocytic leukemia, basal cell cancer, ovarian epithelial cancer, ovarian germ cell cancer, breast cancer, brain cancer, pituitary adenoma, multiple myeloma, gallbladder cancer, biliary tract cancer, large intestine cancer, chronic myeloid leukemia, chronic lymphocytic leukemia, retinoblastoma, choroidal melanoma, ampulla of Vater cancer, bladder cancer, peritoneal cancer, parathyroid cancer, adrenal cancer, sinonasal cancer, non-small cell lung cancer, tongue cancer, astrocytoma, small cell lung cancer, pediatric brain cancer, pediatric lymphoma, pediatric leukemia, small intestine cancer, meningioma, esophageal cancer, glioma, renal pelvis cancer, kidney cancer, heart cancer, duodenum cancer, malignant soft tissue cancer, malignant bone cancer, malignant lymphoma, malignant mesothelioma, malignant melanoma, eye cancer, vulvar cancer, ureteral cancer, urethral cancer, cancer of unknown primary site, gastric lymphoma, stomach cancer, gastric carcinoid, gastrointestinal stromal cancer, Wilms cancer, breast cancer, sarcoma, penile cancer, pharyngeal cancer, gestational trophoblastic disease, cervical cancer, endometrial cancer, uterine sarcoma, prostate cancer, metastatic bone cancer, metastatic brain cancer, mediastinal cancer, rectal cancer, rectal carcinoid, vaginal cancer, spinal cord cancer, acoustic neuroma, pancreatic cancer, salivary gland cancer, Kaposi's sarcoma, Paget's disease, tonsil cancer, squamous cell carcinoma, lung adenocarcinoma, lung cancer, squamous cell carcinoma of the lung, skin cancer, anal cancer, rhabdomyosarcoma, laryngeal cancer, pleura cancer, and thymic cancer.

11. The pharmaceutical composition according to claim 7, wherein the compound inhibits EGFR (epidermal growth factor receptor) and HER2 mutations.

12. The pharmaceutical composition according to claim 7, wherein the anticancer effect is enhanced by administration of the pharmaceutical composition in combination with an anticancer agent.
